# EUROPEAN PATENT SPECIFICATION

(11) **EP 2 900 319 B1**
(45) Date of publication and mention of the grant of the patent: **19.04.2017**
(21) Application number: 13770478.9
(22) Date of filing: 26.09.2013
(51) Int. Cl.: A61N 1/375, H05K 5/06

(54) **IMPLANTABLE DEVICES**
IMPLANTIERBARE VORRICHTUNGEN
DISPOSITIFS IMPLANTABLES

(30) Priority: 28.09.2012 US 201261707473 P
(43) Date of publication of application: 05.08.2015
(73) Proprietor: CSEM Centre Suisse d'Electronique et de Microtechnique SA - Recherche et Développement, 2007 Neuchâtel (CH)
(72) Inventor: JOSE JAMES, Rony, 6055 Alpanach Dorf (CH); SPINOLA DURANTE, Guido, 6003 Luzern (CH); BOSSHARD, Christian, 4415 Lansen (CH)
(74) Representative: Gevers SA
(86) International application number: PCT/EP2013/070130
(87) International publication number: WO 2014/049089

(56) References cited:
- WO-A2-2012/015756
- US-A- 5 738 270
- US-A1- 2003 071 283
- A. FAN ET AL: "Copper Wafer Bonding", ELECTROCHEMICAL AND SOLID-STATE LETTERS, vol. 2, no. 10, 1 January 1999 (1999-01-01), page 534, XP055088560, ISSN: 1099-0062, DOI: 10.1149/1.1390894

## Description

### Technical Field

The present description relates to the field of implantable devices, in particular implantable medical devices comprising an electronic circuit, suitable for long-term implantation in the body of a patient. The present invention is set out in the appended claims.

### State of the art

Conventionally, packaging of active implantable medical devices mainly involves the hermetic sealing of active circuitry inside a package and feedthroughs to transfer Input/Output (I/O) signals to the outside of the package. The electronics unit is typically packaged in a titanium housing that is hermetically sealed using laser welding. The electrical feedthroughs to transfer Input/Output (I/O) signals to the outside of the package are based on the use of metal pins inserted in a biocompatible ceramic shield. These metal pins are typically made of platinum-iridium or niobium. The bonding between the pins and the ceramic is made using a biocompatible filler material or by co-firing the pins with the ceramic in the green state. This feedthrough is then bonded to a titanium flange (typically Au brazing) for further welding to the hermetic titanium box. With the above technologies, the miniaturization is limited: (i) Laser welding has a limitation for miniaturization due to the heated zone when miniaturizing the packages (lateral sizes down to a few mm). (ii) The miniaturization of the feedthroughs is limited and is always depending on the parameters such as the size of the pin, the distance between the holes and the size of each of the sub-parts (titanium flange, ceramic substrate and pin). The minimum feedthrough size commercially available to the best of our knowledge has a dimension of 3.8 mm.

Furthermore, due to the heating requirement, it can be very difficult to achieve CMOS-compatible manufacturing processes, due to the lack of heat tolerance of CMOS devices, which must be kept below about 250° C.

Yet furthermore, typical feedthroughs made with current technology typically require biocompatible material to be used to fill the via, which can be expensive.

Prior art examples of implantable devices include those disclosed in the following documents: US 6,472,112, US 2010/0272208, US 2008/0102096, WO 2007/118133, US 2011/0125210, EP 2 021 757 and US 7,079,900. Furthermore, WO2012/015756 discloses an implantable device according to the preamble of claim 1; US 5,738,270 discloses the preamble of claim 8; "Copper Wafer Bonding" to Fan et al. (Electrochemical and Solid-State Letters, 2 (10) 534-536 (1999)) discloses a bonding technique based on copper metallization for implementing 3-D integrated circuits; and US2003/0071283 describes a semiconductor structure for optoelectronic devices.

The aim of the description is thus to overcome at least partially the above-mentioned limitations of the prior art.

### Disclosure of the invention

More particularly, an objective of the present description is to provide solutions for miniaturized, long term biocompatible hermetically sealed packages to be used in active implantable devices. Applications for these implantable devices include, but are not limited to, spinal cord stimulation, deep brain stimulation, sacral nerve stimulation, cochlear implants, orthopedic monitoring, optical neurostimulation, optoacoustic stimulation, optical pacemakers, (implantable) microphones, retina prosthesis, glucose monitoring, optical imaging and management of cardiovascular diseases and other similar active implantable medical devices.

This object is attained by an implantable device comprising a base element, which may be simply a substrate, a feedthrough element, or similar, and a cover attached to said base element so as to define a cavity arranged to receive an electronic device. A metallic interlayer is disposed between the base element and the cover, arranged so as to bond the base element to the cover. The metallic interlayer comprises at least one diffusion barrier layer, which may also constitute an adhesion layer for assuring good adhesion between the metallic interlayer and the underlying material, and at least one bonding layer. Naturally, all the materials destined to be in contact with bodily fluids should be biocompatible, according to the appropriate medical standards. It should be noted that a further, dedicated, adhesion layer may be situated between the diffusion barrier layer and the cover or the base element, such a dedicated adhesion layer being for instance a titanium-comprising layer.

In consequence, due to the combination of a diffusion barrier layer and a bonding layer, relatively high temperature bonding processes can be used when fabricating the implantable device, nevertheless assuring CMOS compatibility since the electronic device does not get too hot during assembly of the implantable device thanks to the localization of the heating, without material of any additional adhesion layer, of the base, or of the cover contaminating the bonding layer by diffusion, or vice versa, thereby ensuring excellent bonding. Indeed, the diffusion barrier prevents that some of the material of the bonding layer reaches the interface between the layer that serves as adhesion layer and the base element -- or the cover, as appropriate - which would weaken the bonding. In the case where there is a dedicated adhesion layer, the diffusion barrier also prevents the material of the adhesion layer from reacting with the bonding layer and from weakening the bonding.

Advantageously, the base element may be a feedthrough element comprising a substrate provided with at least one electrical contact element disposed on a first side of the substrate, said electronic device being in electrical connection with said electrical contact element.

Advantageously, the said feedthrough element may comprise an intermediate wall element, said cover being attached to said intermediate wall element, said metallic interlayer being disposed between the cover and the intermediate wall element, and wherein a further metallic interlayer, which may be the same or different to that above, is preferably disposed between the intermediate wall element and the substrate.

Advantageously, the cover may be substantially transparent, facilitating e.g. laser welding of the metallic interlayers through the cover, and/or permitting light to enter or leave the implantable device. To this end, the implantable device may further comprise at least one lens integral with, or attached to, said cover.

Advantageously, the implantable device may further comprise an further electrically nonconductive substrate bonded to said feedthrough element and extending laterally therefrom, said cover extending laterally from the feedthrough element and substantially parallel to said further electrically nonconductive substrate , and further comprising at least one electrical interconnection and a deformable elastic element sandwiched one above the other between said cover and said further electrically nonconductive substrate. A particularly compact and effective arrangement for interconnections between individual implantable devices is thereby possible. The deformable elastic element may be formed as a strand, a wire, or a layer, and/or the electrical interconnection may comprise a deformable elastic wire wound with at least one electrically conducting filament.

Advantageously, the implantable device may further comprise at least one electrical line at least partially disposed between said feedthrough element and said further electrically nonconductive substrate , said electrical line being in electrical communication with at least one electrical contact element, and said electrical interconnection being in electrical communication with said at least one electrical line. Compact and rugged electrical connections between individual implantable devices, or between implantable devices and other devices are thus possible.

Advantageously, the diffusion barrier layer may comprise tantalum, and the bonding layer may comprise platinum.

Advantageously, the metallic interlayer may further comprise at least one absorption layer adapted to absorb laser light, which permits efficient laser welding and/or efficient laser-assisted diffusion bonding. The absorption layer may comprise titanium. The absorption layer may also serve as an adhesion layer, or alternatively a further, dedicated adhesion layer may be provided between the absorption layer and the material of the surface upon which it is situated.

Advantageously, the metallic interlayer may comprise at least once the sequence of layers tantalum - platinum, preferably the sequence of layers tantalum - platinum - tantalum.

Alternately, the metallic interlayer may comprise the sequence of layers titanium - tantalum - platinum, preferably the sequence of layers titanium - tantalum - platinum - tantalum - titanium.

Advantageously, the cover comprises an antenna on a surface thereof, preferably an inwardly-facing surface. The antenna may be configured to improve wireless communication to the electronic device. The antenna may also be configured as a remote powering antenna that can be used either to continuously power the electronic device, or to recharge a battery or capacitor which would be located inside a package. The antenna may also be configured to improve wireless communication and at the same time as a remote powering antenna.

The object of the description is also attained by a method of manufacturing an implantable device as defined above, the method comprising the steps of:
- providing a base element comprising a substrate;
- providing a cover;
- forming a metallic interlayer on at least one of said feedthrough element and said base element, said metallic interlayer comprising at least one diffusion barrier layer, which may serve as an adhesion layer or a separate dedicated adhesion layer may be provided, and at least one bonding layer;
- bonding the cover to the base element by means of at least one of:
   - diffusion bonding;
   - laser assisted diffusion bonding;
   - laser welding;
   - thermosonic welding;
   - ultrasonic welding.

The use of the diffusion barrier layer (possibly serving as adhesion layer) and the bonding layer in the metallic interlayer permits the use of the listed bonding/welding techniques, which, on the one hand, ensure the CMOS compatibility of the manufacturing method due to the fact that an electronic device disposed inside the implantable device will not be subjected to high temperatures during manufacture of the implantable device thanks to the localization of the heating, and which, on the other hand, prevents any material of the bonding layer to weaken the bond between the layer that serves as adhesion layer and the base elements (or the cover, as appropriate). In the case where there is a dedicated adhesion layer, the diffusion barrier also prevents the material of the adhesion layer from reacting with the bonding layer and from weakening the bonding.

Advantageously, the base element may be a feedthrough element comprising a substrate provided with at least one electrical contact element formed on a first side thereof, said electronic device being in electrical connection with said electrical contact element. The method may further advantageously comprise forming a respective metallic interlayer on each of said base element and said cover, ensuring excellent bonding between these parts. The metallic interlayer may be advantageously formed by depositing a diffusion barrier layer, and then depositing a bonding layer on the diffusion barrier layer. A dedicated adhesion layer may also be formed before formation of the diffusion barrier layer, the diffusion barrier layer being formed thereupon.

Advantageously, the diffusion barrier layer may comprise tantalum, and/or said bonding layer may comprise platinum.

Advantageously, the method may further comprise depositing an absorption layer for the absorption of laser light before depositing said diffusion barrier layer, said absorption layer preferably comprising at least titanium. The light absorption layer may also serve as an adhesion layer for improving adhesion between the metallic interlayer and the underlying material..

Advantageously, the method may further comprise attaching a further electrically nonconductive substrate to said electrical contact elements. This cover may extend laterally from the feedthrough element substantially parallel to the further electrically nonconductive substrate, the method further comprising placing at least one electrical interconnection and a deformable elastic element sandwiched one above the other between said cover and said further electrically nonconductive substrate and compressing said deformable elastic element upon bonding of the cover to the feedthrough element. Compact and rugged electrical connections between individual implantable devices can thus be made.

Advantageously, the method may further comprise providing at least one electrical line upon said further electrically nonconductive substrate, said electrical line being in electrical communication with said contact elements, said electrical interconnection being in electrical communication with said at least one electrical line.

The object of the description is also attained by a feedthrough element for an implantable device such as those disclosed above, or for any other type, comprising :
- a substrate comprising at least one feedthrough opening, said feedthrough opening being hermetically closed on a first side of said substrate by a metal contact element having a width greater than the width of the respective feedthrough opening, and said feedthrough opening being filled with an electrically conductive material ;
- a metallic interlayer bonding said metal contact element to said substrate around the circumference of said feedthrough opening, said metallic interlayer comprising at least one diffusion barrier layer and at least one bonding layer. The diffusion barrier layer may constitute an adhesion layer, or a further, dedicated, adhesion layer may be formed on the diffusion barrier layer, e.g. a titanium-containing layer

As for the implantable device itself above, due to the combination of a diffusion barrier layer and a bonding layer, relatively high temperature bonding processes can be used when fabricating the implantable device without excessively heating any electronic device present, thereby assuring CMOS compatibility, without the material of the base or of the cover contaminating the bonding layer by diffusion, or vice versa, ensuring excellent bonding. Indeed, the diffusion barrier prevents that some of the material of the bonding layer reaches the interface between the layer that serves as adhesion layer and the base element -- or the cover, as appropriate - which would weaken the bonding. In the case where there is a dedicated adhesion layer, the diffusion barrier also prevents the material of the adhesion layer from reacting with the bonding layer and from weakening the bonding. Furthermore, such an arrangement permits the use of non-biocompatible, and hence cheaper and potentially easier to work with, material for filling the feedthrough, since it will not come in contact with bodily fluids, since it is hermetically sealed therefrom by the other components of the feedthrough and of the resulting implantable device to which the feedthrough is assembled. Yet further, this geometry permits a relatively high density of feedthroughs to be constructed, and for the substrate to be relatively thin, permitting a very compact construction and consequent possibilities for miniaturisation.

Advantageously, the metal contact element may be a metal foil contact element.

Advantageously, the feedthrough element may further comprise a peripheral metal element, preferably a metal foil element, forming a flange protruding from the periphery of the substrate, preferably arranged on said first side of said substrate, and a further metallic interlayer bonding the peripheral metal element to said substrate. This peripheral metal element may for instance be formed at the same time as the metal contact elements, and serves as a convenient attachment point for a cover or similar.

Advantageously, the substrate may comprise a base portion, and a sidewall portion substantially perpendicular to the base portion.

Advantageously, the metal contact element may constitute at least one of: an electrical pad; an electrical track.

Advantageously, the feedthrough element may further comprise a flange attached to the periphery of the substrate.

Advantageously, the feedthrough element may further comprise a further electrically nonconductive substrate bonded to said substrate. Said nonconductive substrate may prevent the metal contact elements, once implanted in the body, to be in electrical contact with the body, or between themselves when several are present, since body liquid is electrically conductive.

Advantageously, the diffusion barrier layer may comprise tantalum, and/or said bonding layer may comprise platinum.

Advantageously, the metallic interlayer may further comprise at least one absorption layer adapted to absorb laser light, which may also serve as an adhesion layer. Alternatively, a dedicated adhesion layer may also be formed between the substrate and the absorption layer.

Advantageously, the absorption layer may comprise titanium.

Advantageously, the metallic interlayer may comprise the sequence of layers tantalum - platinum, preferably the sequence of layers tantalum - platinum - tantalum.

Advantageously, the metallic interlayer may comprise the sequence of layers titanium - tantalum - platinum, preferably the sequence of layers titanium - tantalum - platinum - tantalum - titanium.

Additionally, the object of the description is also attained by an implantable device comprising :
- a feedthrough element as defined above;
- a cover attached directly or indirectly to said feedthrough element so as to define a hermetically sealed cavity; and
- an electronic device disposed in said cavity in electrical connection with said metal contact element.

Advantageously, the cover is attached to the substrate of the feedthrough element.

Advantageously, the implantable device may further comprise a feedthrough element incorporating a peripheral metal element as described above, wherein said cover is attached to said peripheral metal element.

Advantageously, the feedthrough element may comprise an intermediate wall element disposed between the cover and the substrate, said cover being attached to said intermediate wall element.

The object of the description is also attained by a method of manufacturing a feedthrough element as defined above, the method comprising the steps of:
- providing a substrate;
- forming at least one metallic interlayer upon at least part of said substrate, said metallic interlayer comprising at least one diffusion barrier layer and at least one bonding layer;
- structuring said substrate so as to form at least one feedthrough opening;
- forming a metal contact element so as to close said feedthrough opening, said metal contact element having a width greater than the width of said feedthrough opening;
- filling said feedthrough opening with an electrically conductive material, wherein said metal contact element is bonded to the substrate by means of said metallic interlayer, said bonding being carried out by at least one of:
   - diffusion bonding;
   - laser assisted diffusion bonding;
   - laser welding;
   - thermosonic welding;
   - ultrasonic welding.

As for the method of manufacturing an implantable device above, the use of the diffusion barrier layer and the bonding layer in the metallic interlayer permits the use of the listed bonding/welding techniques, which ensure the CMOS compatibility of the manufacturing method due to the fact that an electronic device disposed inside the implantable device will not be subjected to high temperatures during manufacture of the implantable device. Furthermore, such a method permits the use of non-biocompatible, and hence cheaper and potentially easier to work with, material for filling the feedthrough, since it will not come in contact with bodily fluids, due to being hermetically sealed therefrom by the other components of the resultant feedthrough and of the resulting implantable device to which the feedthrough is assembled. Yet further, this method permits a relatively high density of feedthroughs to be constructed, and for the substrate to be relatively thin, permitting a very compact construction of the resulting feedthrough element and consequent possibilities for miniaturisation.

Advantageously, the metal foil contact element may be formed by:
- providing a sheet of metal foil;
- bonding said sheet of metal foil to said substrate by means of said metallic interlayer;
- structuring the sheet of metal foil to form at least one electrical pads and/or at least one electrical track.

In consequence, since the sheet of metal foil can easily be microstructured e.g. by laser, lithography or similar, relatively small metal foil contact elements can be produced at relatively high density, leading to a high degree of miniaturisation of the resulting feedthrough element.

Advantageously, the method may further comprise forming a flange protruding from the periphery of the substrate, said flange comprising a peripheral metal foil element and preferably being arranged on said first side of said substrate.

Advantageously, the flange is formed by structuring said sheet of metal foil, permitting both the flange and the metal contact elements to be formed simultaneously in a single step, leading to efficient production.

Additionally, the object of the description is attained by a method of manufacturing an implantable device comprising a feedthrough element as defined above, the method comprising the steps of:
- manufacturing a feedthrough element by any of the appropriate methods above;
- installing an electronic device in electrical connection with said at least one metal foil contact element;
- hermetically attaching a cover directly or indirectly to said feedthrough element so as to define a hermetically sealed cavity containing said electronic device.

Advantageously, the cover may be attached by providing a metallic interlayer on at least one of the cover and the feedthrough element or an intermediate element attached to the feedthrough element, subsequently bonding the cover by means of at least one of:
- diffusion bonding;
- laser assisted diffusion bonding;
- laser welding;
- thermosonic welding;
- ultrasonic welding.

Advantageously, the method may further comprise attaching an electrically-nonconductive base element to said metal foil contact elements.

Advantageously, the metallic interlayer may be formed by depositing directly or indirectly a diffusion barrier layer on at least part of the substrate, then depositing a bonding layer on the diffusion barrier layer. The diffusion barrier layer may comprise tantalum, and/or the bonding layer may comprise platinum. The diffusion barrier layer may serve as an adhesion layer, or alternatively a dedicated adhesion layer, e.g. containing titanium, may be formed between the diffusion barrier layer and the cover or feedthrough element, as appropriate.

Advantageously, the method may further comprise depositing an absorption layer on said substrate before depositing said diffusion barrier layer on said absorption layer, said absorption layer preferably comprising titanium. This absorption layer may also constitute an adhesion layer for improving adhesion between the metallic interlayer and the underlying material of the surface upon which it is formed.

It should be noted that all of the feedthrough elements described above may be applied to all of the implantable devices as described above.

Finally, the object of the description is attained by the use of an implantable device according to the present description for one of the following:
- spinal cord stimulation;
- deep brain stimulation;
- sacral nerve stimulation;
- orthopaedic monitoring;
- glucose monitoring;
- optical neurostimulation;
- optical imaging;
- management of cardiovascular disease;
- as a cochlear implant;
- as an implantable microphone;
- as a retinal prosthesis;
- as an optical pacemaker.

Naturally, it is noted that with such uses the implantable device will further comprise appropriate electronic circuitry therewithin.

It should also be noted that all advantageous features may be combined in any combination, so long as such a combination is not self-contradictory.

### Brief description of the drawings

Further details of the description will now be explained in reference to the following figures, which show schematically:
- Figures 1a, 1b: cross-sectional views of general geometries of implantable devices;
- Figure 2: a cross-sectional view of a further geometry of an implantable device;
- Figure 3: a cross-sectional view of the general geometry of a MEMS microphone;
- Figure 4: a cross-sectional view of two parts joined by metallic interlayers;
- Figure 5: a cross-sectional view of a further example of two parts joined by metallic interlayers;
- Figure 6: a cross-sectional view of a further example of two parts joined by metallic interlayers;
- Figure 7: a cross-sectional view of a further example of two parts joined by metallic interlayers;
- Figure 8: a cross-sectional diagram of forces acting in a welding zone during ultrasonic bonding;
- Figure 9: a cross-sectional diagram of ultrasonic bonding of an implantable micro package;
- Figure 10: a cross-sectional diagram of laser welding;
- Figure 11: a further cross-sectional diagram of laser welding;
- Figure 12: a further cross-sectional diagram of laser welding for thermally matched substrates;
- Figures 13a, b: cross-sectional diagrams of laser welding of an implantable device with a metal preform as a cover;
- Figure 14: a further cross-sectional diagram of laser welding an implantable MEMS microphone;
- Figure 15: a cross-sectional diagram of laser-assisted diffusion bonding;
- Figure 16: a cross-sectional diagram of laser-assisted diffusion bonding as applied to thermally-matched substrates;
- Figure 17a: a cross-sectional view of an embodiment of a feedthrough element;
- Figure 17b: a cross-sectional view of an embodiment of an implantable device comprising the feedthrough element of figure 17a.
- Figure 18: a cross-sectional view of a further embodiment of a feedthrough element;
- Figure 19a: a cross-sectional view of a yet further embodiment of a feedthrough element;
- Figure 19b: a cross-sectional view of the feedthrough element of figure 19a applied to an implantable device;
- Figure 20: a cross-sectional view of an implantable device based on flip-chip bonding;
- Figure 21: a cross-sectional view of an implantable device based on wirebonding;
- Figures 22a-c: bottom views of various feedthrough geometries;
- Figure 23: a cross-sectional view of a further embodiment of an implantable device based on flip-chip bonding;
- Figure 24: a cross-sectional view of a further embodiment of an implantable device based on wirebonding;
- Figure 25: a bottom view of a feedthrough geometry of the devices of figures 23 and 24;
- Figures 26a, b: cross-sectional and bottom views of further embodiments of feedthrough devices;
- Figure 27: a cross-sectional diagram of diffusion bonding;
- Figure 28: a cross-sectional diagram of laser-assisted diffusion bonding;
- Figure 29a: a cross-sectional diagram of diffusion bonding using a thin metal film;
- Figure 29b: a cross-sectional diagram of laser-assisted diffusion bonding using a thin metal film;
- Figure 30: a flow diagram of a most generic method of manufacturing a feedthrough element;
- Figure 31: a flow diagram of a most generic method of manufacturing an implantable device;
- Figure 32: a flow diagram of an example of a more detailed method for manufacturing an implantable device;
- Figures 33a, b: cross-sectional views of chip on wire assembly;
- Figure 34: a side view of an array of chips assembled using chip on wire technologies;
- Figure 35: a cross-sectional view of a further embodiment of chip on wire assembly;
- Figure 36: a cross-sectional view of a further embodiment of chip on wire assembly;
- Figure 37: a cross-sectional view of an implantable light source;
- Figure 38: a cross-sectional view of another embodiment of an implantable light source;
- Figure 39a, b: cross-sectional views of another embodiment of an implantable light source;
- Figure 40: a cross-sectional view of an implantable vision sensor;
- Figure 41: a cross-sectional view of an intelligent electrode;
- Figure 42: a cross-sectional view of a combined intelligent electrode and light source micro package;
- Figure 43: a perspective view of an optical neurostimulation system;
- Figure 44: a cross-sectional view of packaged light sources and corresponding electrodes;
- Figure 45a: a cross-sectional view of an implantable device optimized for wireless applications;
- Figure 45b: a view of the inside of the cover of the implantable device of figure 45a.

### Embodiments of the description

The present invention is set out in the appended claims. The embodiments, aspects or examples according to the present description that do not fall within the scope of said claims are provided for illustrative purposes only and do not form part of the present invention.

### Materials

Suitable materials for implantable devices that are exposed to the human body must have been proven to be long term biocompatible, long-term implantable, and must provide a hermetic barrier. They include, but are not limited to, platinum, titanium, alumina, sapphire, zirconia, tantalum, nitinol, niobium, some platinum iridium alloys and some cobalt chromium alloys. These materials can be used in any appropriate combinations. In addition, although not yet proven to be long-term implantable, materials such as fused silica, gold, silver and palladium are considered as well. Flexible biocompatible and longterm implantable materials suitable for chip-on wire technologies include biocompatible polyurethane and silicone elastomers and duromers.

However, it should be noted that the materials described above are not be considered as limiting.

### General implantable device geometries

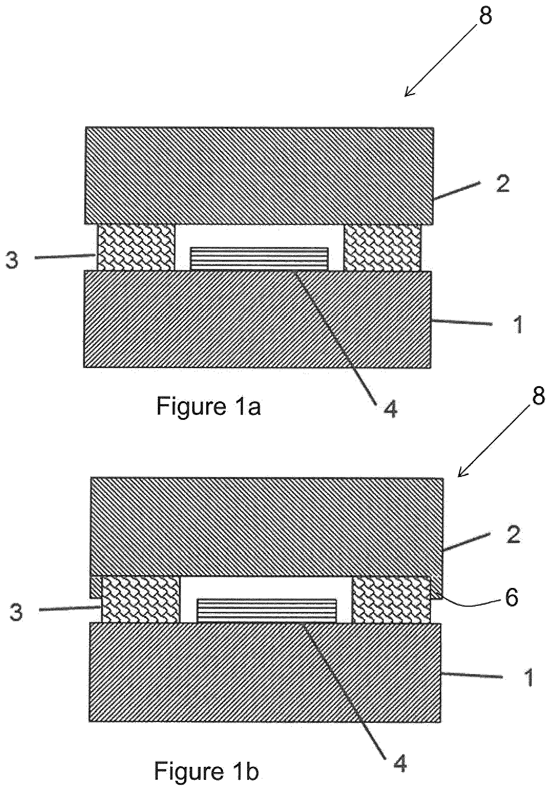

The basic geometry of an implantable device 8, also here interchangeably referred to as a "micropackage", is shown in Figures 1a and 1b. The device 8 comprises a biocompatible, long-term implantable base 1, in this embodiment being constituted solely by a substrate 1, and biocompatible, long-term implantable cover 2, referred to interchangeably as a "cap". Base 1 would usually be a feedthrough element (see below), although this does not have to be the case. For clarity, the bonds of the active circuit in the package, the electrical interconnections and electrically conductive vias, if present, are not shown in Figure 1. Substrate 1 would typically be of a ceramic substance, whereas cover 2 may be any implantable substance such as a ceramic or a metal, e.g. in the form of a metal foil. The hermetic sealing is provided through biocompatible, long-term implantable metal interlayers 3. These metal interlayers always refer to combinations of (or parts of combinations of) titanium, platinum, tantalum and niobium (all approved for long-term implantability) of various thicknesses. It should be noted for completeness that the metal interlayers can also be completely absent, the cover 2 and the base 1 being bonded directly to one another e.g. by ultrasonic welding. Other metals like silver and gold could be used as well if proven to be long-term biocompatible. The metals can be deposited using combination of methods including evaporation, sputtering and electroplating. For all geometries described herein the metal interlayers 3 can vary between different bonds in terms of material selection, thickness, area, and position in/on the implantable device. This means for example that also within one implantable device 8, metal interlayers 3 can vary from one bond to another, e.g. the metal interlayers can be different for the hermetic seal of the devices 8 and the hermetic seal for the feedthroughs. Inside the device 8, there is an active circuit 4. An active circuit means one or more active (including e.g. (combinations of) application-specific integrated circuits (ASIC), vision chips, (wireless) communication chips, microelectromechanical systems (MEMS), Microopto-electromechanical systems (MOEMS), batteries) or passive components.

The device 8 can have any shape, e.g. it can be rectangular, round or ellipsoidal or another shape. In some of the figures below for simplicity the substrate and the cover of the device have the same surface area. In general, the surface areas, thicknesses, and shapes of the various components of the device can be different (see e.g. also in Figures 1a, 1b). For instance, the structure of figure 1a comprises a planar cover 2, whereas the structure of figure 1b comprises a cover 2 with a flange 6 extending towards the base 1 around the periphery of the cover 2.

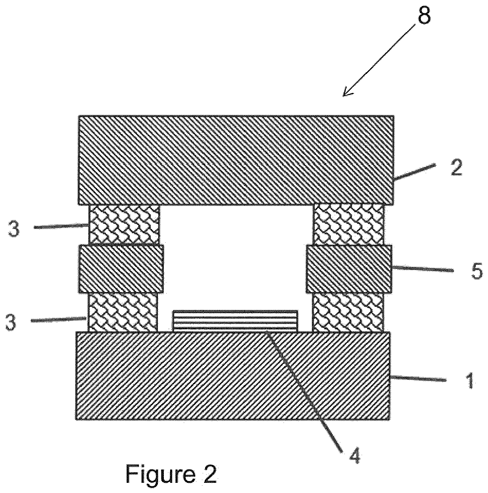

Figure 2 shows another example of a general geometry of an implantable device 8. The device 8 comprises a biocompatible, long-term implantable base 1 and biocompatible, long-term implantable cover 2. The hermetic sealing is provided through biocompatible, long-term implantable metal interlayers 3 and biocompatible, long-term implantable bonding ring frames 5, forming intermediate wall elements. This bonding ring frame 5 may be formed e.g. by a ceramic preform, or a metal preform. The metal interlayers 3 above and below the ring frame can be the same or different. Also more than one ring frame 5 and more than two metal interlayers 3 can be used as well. Inside the device 8 is an active circuit 4. Again, for clarity, the electrical interconnections and electrically conductive vias are not shown.

In all of the above cases, if the active circuit 4 can communicate wirelessly and be powered wirelessly, there is no requirement for the base 1 to comprise feedthroughs, and the base 2 may in this case simply be a single piece.

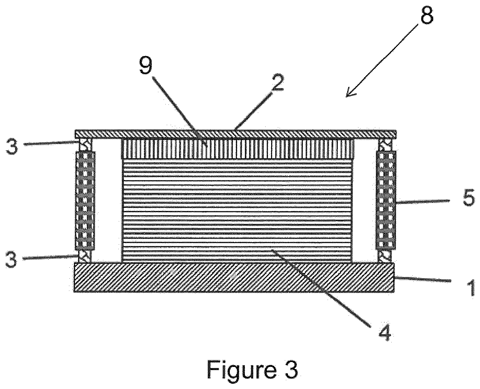

Figure 3 shows a general geometry of an example of an implantable device 8 for a MEMS device (in this case a microphone 4). Generally, any MEMS device (e.g. MEMS microphones and pressure sensors) can be packaged in an analogous way. The device 8 comprises a biocompatible, long-term implantable base 1 and biocompatible, long-term implantable membrane as cover 2, which may be made of materials such as Ti and Sapphire. The hermetic sealing is provided through biocompatible, long-term implantable metal interlayers 3 and biocompatible, long-term implantable bonding ring frames or walls 5. Inside the device 8 is a microphone 4 (with an additional active circuit, if needed). In between the membrane 2 and the microphone 4 a (soft) layer 9 for optimizing the sound transfer can be added. As another example, in case of a pressure sensor the layer between the pressure sensor membrane and the sealing membrane would help to optimize the pressure transfer between them. For clarity, the electrical interconnections and electrical feedthroughs are not shown. The membrane 2 is hermetically bonded to the wall 5. The walls can be bonded to the substrate of the base 1. Alternatively, the base 1 and the walls 5 can be fabricated as one piece.

In case a wireless connection is required, an antenna (not illustrated) can directly be incorporated into the device 8 as the walls 5 are nonconductive. The communication module can then be part of the active circuit 4 and can be connected to the antenna with standard processes including wirebonding and flip-chip bonding.

### Hermetic sealing of implantable devices

In the following is described the hermetic sealing processes that can be applied to arrive at an implantable device according to the description, namely:
ultrasonic bonding, laser welding, laser-assisted diffusion bonding. Various levels of polishing of the different parts to be bonded may be needed to achieve the hermetic sealing. Typically but not exclusively, the polishing would take place before the metal coating.

Bonding processes that have been described in other patents and publications include fusion bonding, diffusion bonding, anodic bonding, reactive metal film bonding, laser brazing, solder bonding, silicide direct bonding, and eutectic alloy bonding.

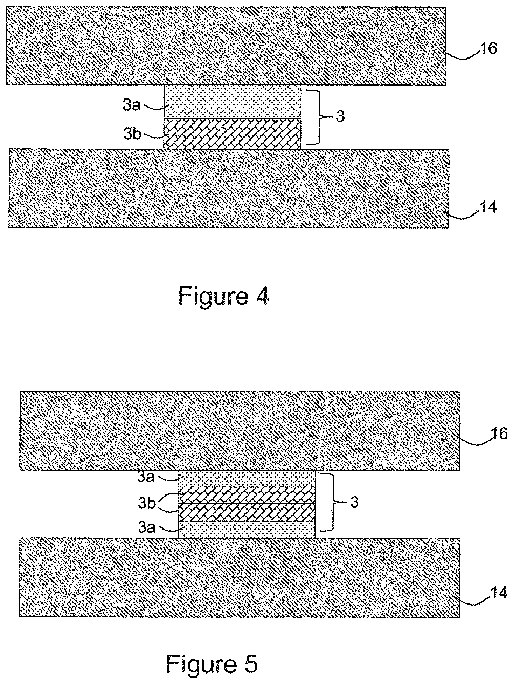

Firstly, figures 4-7 illustrate various structures of the metallic interlayer 3 bonding a first part 14 (such as a base) of an implantable device to a second part 16 (such as a cover) of the implantable device. It should be noted however that the base and the cover are merely named for illustrative purposes, and that the metallic interlayer 3 can likewise be utilised between other components, such as between base and a metal contact element, or any other two components.

In figure 4, metallic interlayer 3 comprises a diffusion barrier layer 3a in contact with a second part 16, and a bonding layer 3b in contact with a first part 14. First part 14 and second part 16 may be any part, such as a substrate, cover, intermediate wall element, feedthrough element or similar. This metallic interlayer structure 3 would be appropriate for the case in which bonding layer 3b can be bonded directly to the material of the first part 14, for instance if the first part 14 is made of platinum or any other suitable metal. Diffusion barrier layer 3a typically comprises tantalum or an alloy thereof, and bonding layer 3b typically comprises platinum or an alloy thereof. Naturally, if appropriate, the sequence of diffusion barrier layer 3a and bonding layer 3b can be reversed. It is clear that, since the diffusion barrier layer 3a is formed in contact with the second part 16, it acts as an adhesion layer. Alternatively, a further adhesion layer (not illustrated) may be formed between second part 16 and diffusion barrier layer 3a if required to improve the adhesion between the metallic interlayer 3 and the second part 16.

Figure 5 illustrates the situation in which the materials of both the first part 14 and the second part 16 are not suitable to be bonded directly to either the first part 14 or the second part 16. This situation arises e.g. if both parts are made of ceramic. In this case, each part 14, 16 is provided with a diffusion barrier layer 3a on the part, and a bonding layer 3b. The bonding layers 3b are then bonded together. The above comments regarding adhesion layers apply equally here.

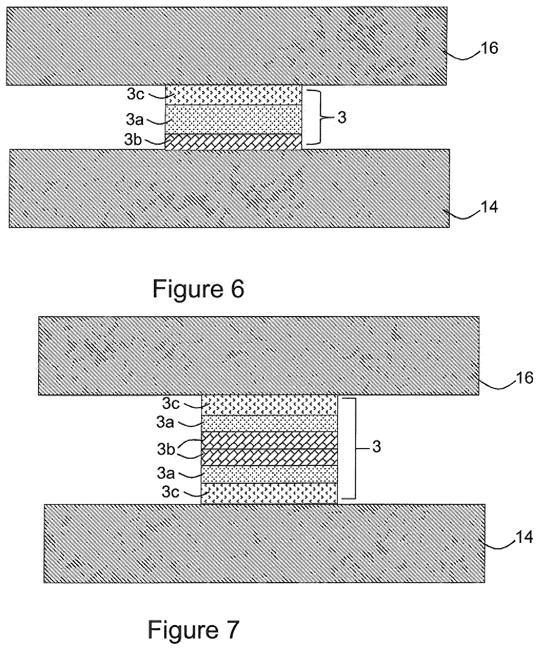

In figure 6, analogous to figure 4, metallic interlayer 3 comprises additionally an absorption layer 3c between the diffusion barrier layer 3a and the second part 16. This absorption layer 3c is e.g. made of titanium, and absorbs laser light during the process of laser welding or laser-assisted diffusion bonding (LADB), as will be described later. It is clear that, since the absorption layer 3c is formed in contact with the second part 16, it acts as an adhesion layer. Alternatively, a further adhesion layer (not illustrated) may be formed between second part 16 and absorption layer 3c if required to improve the adhesion between the metallic interlayer 3 and the second part 16.

Figure 7, analogous to figure 5, illustrates the situation in which each part 14, 16 comprises an absorption layer 3c, a diffusion barrier layer 3a and a bonding layer 3b, the bonding layers 3b being bonded together. The above comments regarding adhesion layers apply equally here.

### Ultrasonic/thermosonic bonding

Ultrasonic bonding is a solid state bonding process which is used to typically weld metals or plastic materials. It can also be used to bond metalized materials such as ceramic or glasses. Moreover, in flip-chip bonding applications, ultrasonic bonding has shown to be very useful and fast process to make fine pitch bonds mainly using gold and sometimes copper. The main metals that have so far been used for bonding are gold, aluminium, silver, platinum and copper.

The ultrasonic bonding technology works on the simultaneous application of static pressure and horizontal vibrations to achieve the solid state bonding. The static pressure applied is much smaller than the yield strength of the material. Even though the real mechanism of solid state ultrasonic bonding is still debated, it is thought to be a diffusion bonding process similar to cold welding.

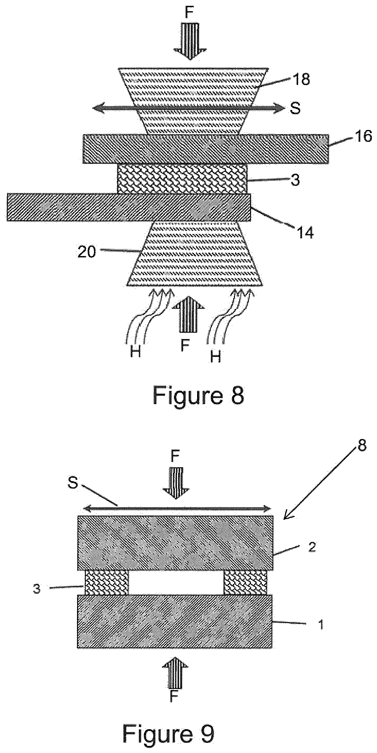

The important parameters for the ultrasonic bonding technology are static force, power, frequency, amplitude of vibration and time. For thermosonic bonding heat is added. Figure 8 shows the forces acting on the welded parts. There are mainly a normal static force F applied between anvil 20 and sonotrode 18, and a shear force S caused by the ultrasonic vibration of the sonotrode acting at the same time on the interface between the respective part 14, 16 and the metallic interlayer 3. As understood by the skilled person, a balance between these two forces is instrumental in achieving a bond. In addition, heat can be applied to improve the bonding process (thermosonic bonding).

Figure 9 shows ultrasonic bonding for the hermetic sealing applied to a general geometry of an implantable device 8. The micropackage comprises a biocompatible, long-term implantable base 1 and biocompatible, long-term implantable cover 2. The hermetic sealing is provided through biocompatible, long-term implantable metal interlayers 3 of the type described above. A normal force F is applied during the bonding process. For clarity, the active circuits, the electrical interconnections and electrically conductive vias are not shown. In addition, heat can be added for the bonding process (thermosonic bonding). The process parameters include, but are not limited to, the range of 200ms-10sec for the bonding time, 1-25kg for the bonding force, vibration frequencies of 5kHz to 200kHz and 1-70 Watt for the ultrasonic power.

### Laser welding

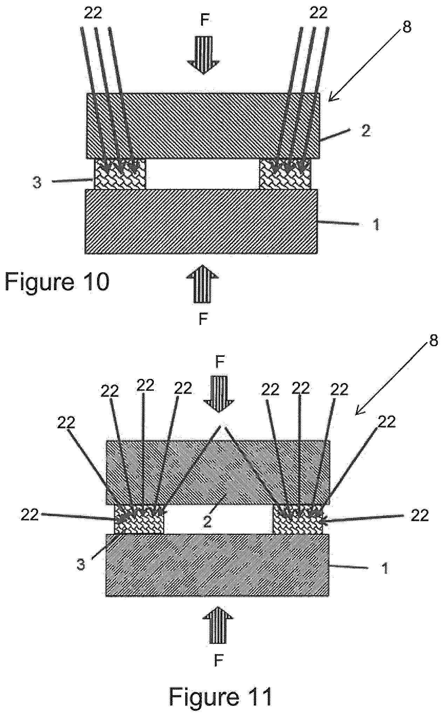

With metal-based laser welding, a laser beam heats up a metal to join parts. It allows to concentrate the heat on a very small spot without affecting the whole sample area. A continuous or pulsed laser beam 22 may be used depending upon the application. The laser must also have an appropriate interaction time to perform the melting process and subsequently the diffusion process to realize a welding spot. Figure 10 shows laser welding for the hermetic sealing applied to a general geometry of implantable micropackages. The micropackage 8 comprises a biocompatible, long-term implantable base 1 and biocompatible, long-term implantable for the laser beam transparent cover 2, e.g. made of alumina, sapphire or similar. The hermetic sealing is provided through biocompatible, long-term implantable metal interlayers 3. A normal force F may be applied during the bonding process. For clarity, the active circuits, the electrical interconnections and electrically conductive vias are not shown. The process parameters may be in the following range: wavelength: from 700nm up to 11µm, pulse duration: from continuous wave to fs-pulse, laser pulse repetition rate: from 0Hz to 1MHz, laser beam area: from 10 micron to 500micron in diameter, laser beam scanning speed: from 0 to 10m/s, laser average power: 1W to 1kW, or even to 10kW nominal force: 5g to 2000g, or even to 20,000g Several laser beam scanning round-trips (1 -10'000) are possible. In another embodiment the laser beam 22 is not scanned and the whole area of the welding zone is illuminated at the same time by a suitable optical system.

Throughout the present description (laser welding, laser-assisted diffusion bonding) the laser beam 22 can be in any direction, i.e. it does not have to be perpendicular to any surface and multiple directions and/or laser beams for one process step are envisioned. This is illustrated schematically in figure 11.

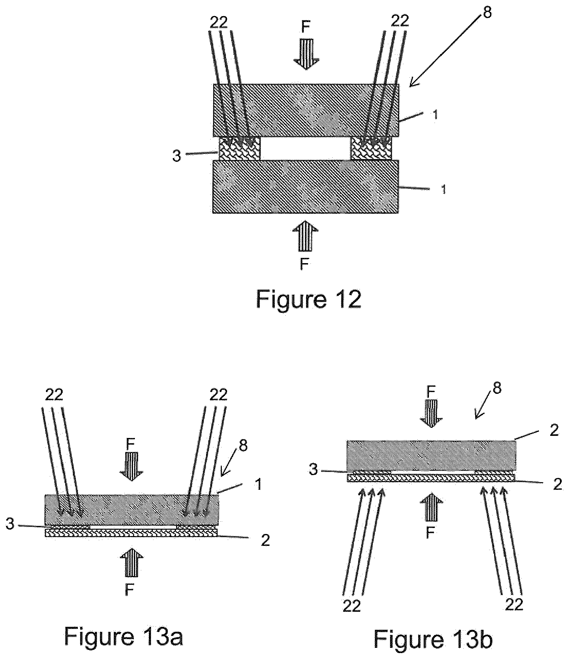

Fig. 12 shows laser welding for the hermetic sealing applied to a general geometry of implantable micropackages for thermally matched substrates. The micropackage comprises a biocompatible, long-term implantable transparent substrate 1 and biocompatible, long-term implantable for the laser beam transparent cover identical to the substrate 1. The hermetic sealing is provided through biocompatible, long-term implantable metal interlayers 3. A normal force F may be applied during the bonding process. For clarity, the active circuits, the electrical interconnections and electrically conductive vias are not shown. The same process parameters as above apply.

In another embodiment, laser welding can be carried out using metal preforms, figures 13a (laser light 22 incident through transparent substrate 1) and 13b (laser light 22 incident on metal preform 2) show a possible implementation. The micropackage comprises a biocompatible, long-term implantable metal preform acting as cap or cover 2 and a biocompatible, long-term implantable substrate acting as base 1. If the laser beam is incident through the substrate it has to be transparent for the laser wavelength used. The hermetic sealing is provided through biocompatible, long-term implantable metal interlayers and/or metal preforms 3. A normal force F is applied during the bonding process. For clarity, the active circuits, the electrical interconnections and electrically conductive vias are not shown. The same process parameters as above apply.

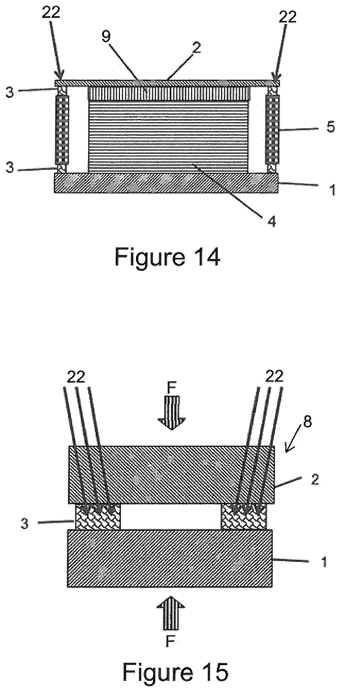

For further illustration, Figure 14 shows the hermetic sealing of a micropackage containing a MEMS device 4, in this case a microphone, and a soft layer 9, analogous to fig. 3 above, using laser welding. The walls can be bonded to the substrate by e.g. laser welding 22, (laser assisted) diffusion bonding (see below), or ultrasonic/thermosonic welding. Alternatively, the substrate and the walls can be fabricated as one piece

### Diffusion bonding

Diffusion bonding is a mechanism where parts to be bonded are brought close to each other and heated to form a bond. For bulk materials bond temperatures as high as half the melting point of the metals are required for these bonds to be reliable. Due to high degree of deformation or/and high degree of disclocation densities, the bond forms at much lower temperatures for thin films or highly reduced materials.

### Laser assisted diffusion bonding

Using a laser to heat the thin film metal surface to high temperatures causes the bond to be formed using very localised heating (also due to the combination of metals (thermally conductive) and dielectrics (thermally poorly conductive) such as e.g. ceramics). In the present description this process is denoted as laser assisted diffusion bonding (LADB). The spot diameters of the laser 22 include the range of 10 um to 10000um which helps to form a highly localised heat affected zone. Indeed, the absorption layer converts the energy from the light beam into heat and is located in a very well controlled position. To realize LADB, metal interlayers 3 (as already described above) for specific laser light absorption, adhesion to substrates, diffusion barrier and diffusion bondability are deposited on a transparent (may be polished) material, e.g. sapphire (cover), single crystalline zirconia which is transparent for typical wavelengths of the laser used and/or on another biocompatible long-term implantable substrate. Cover 2 and substrate 1 (e.g. Sapphire and alumina) are then brought together with force and laser light 22 is used to heat the metal interlayers 3 as shown in Figure 15. Any combinations of cover and substrate using biocompatible and longterm implantable materials are included in the present description. In another embodiment the cover 2 can be a thin metal foil with thicknesses up to 200 microns instead of e.g. Sapphire. Low surface roughness of the samples are critical for a hermetic bonding, as the thin films have much higher yield strength compared to bulk layers and need much more force to deform plastically. Figure 12 shows laser 22 assisted diffusion bonding for the hermetic sealing applied to a general geometry of implantable micropackages 8. The micropackage 8 comprises a biocompatible, long-term implantable base 1 and biocompatible, long-term implantable cover 2 which is transparent to the laser beam 22. The hermetic sealing is provided through biocompatible, long-term implantable metal interlayers 3. A normal force F is applied during the bonding process. For clarity, the active circuits, the electrical interconnections and electrically conductive vias feedthroughs are not shown. Laser assisted diffusion bonding can also be used for all other geometries described, whenever applicable, and it should be noted that the laser beam can also be incident through the substrate if the substrate is transparent. Typical bonding parameters are: wavelength: from 250nm up to 11µm, pulse duration: from continuous wave to fs-pulse (various pulse shapes), laser pulse repetition rate: from 0Hz to 1MHz, laser beam area: from 10 micron to 500micron, or even to 1000micron in diameter, laser beam scanning speed: from 0 to 10m, or even to 50m/s, laser average power: 1W to 1 kW, or even to 10kW. Several laser beam scanning round-trips (1-10'000) are possible. In another embodiment the laser beam is not scanned and the whole area of the welding zone is illuminated at the same time by a suitable optical system. Normal forces are in the range of 5 to 180 MPa and bonding times are in the ms to s range.

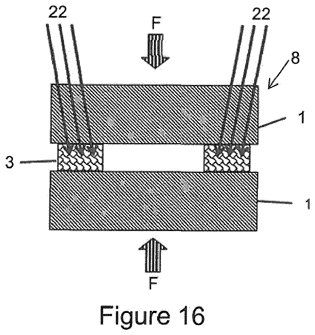

Figure 16 shows laser 22 assisted diffusion bonding for the hermetic sealing applied to a general geometry of implantable micropackages 8 using thermally matched substrates 1. The micropackage 8 comprises a biocompatible, long-term implantable base 1 and biocompatible, long-term implantable for the laser beam transparent cover 2 identical to the substrate 1. The hermetic sealing is provided through biocompatible, long-term implantable metal interlayers 3. A normal force F is applied during the bonding process. For clarity, the active circuits, the electrical interconnections and electrically conductive vias are not shown.

### Feedthrough element

In this section, feedthrough technologies are disclosed that are compatible with a large number of electrically conductive vias that can be closely spaced and are therefore compatible with implantable electrical interconnections. Typical via diameters include the range from 50 microns - 5 mm and the via pitches include values from 60 microns to 5 cm, preferably 60 microns to 5cm. The hermetic sealing is mostly achieved through biocompatible longterm implantable metal pads, wires or foils or ribbons that are bonded to the micropackage with the methods described below. These feedthroughs permit a much higher density of feedthroughs on an implantable device.

The feedthrough element 7 for an implantable device will now be described in more detail. The feedthrough element is the portion of the enclosure of the implantable device which comprises the electrical vias or feedthroughs enabling electrical connection between an electronic circuit located inside the implantable device and the exterior.

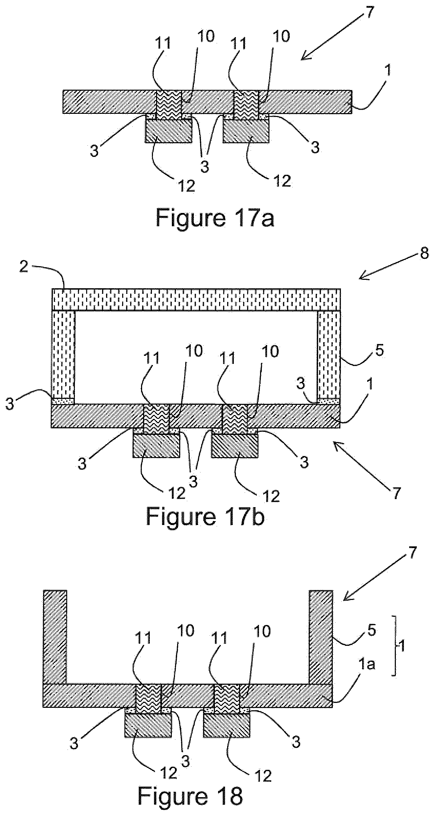

Figure 17a illustrates an embodiment of such a feedthrough element 7, which may form a base of an implantable device of a type described above, or of any other type of implantable device/micropackage.

Feedthrough element 7 comprises a substrate 1, which would typically be of a biocompatible ceramic material. Substrate 1 is traversed by at least one feedthrough opening 10, which is closed on a first side of the substrate 1 (the lower side in the orientation of the figures) by a metal contact element 12, which may be a deposited layer, a foil, or similar. Metal contact element 12 is bonded to substrate 1 by means of metal interlayer 3, of the type disclosed above. Metal contact element 12 may also be bonded to metal interlayer 3 via the intermediary of gold brazing (not illustrated). Feedthrough opening 10, which may be of any convenient shape, e.g. circular, square, oval etc., is filled with a conductive material, so as to form a via permitting electrical connection between an electronic circuit (not illustrated) and metal foil contact element 12.

Since the feedthrough opening 10 is hermetically sealed by means of metal interlayer 3 and metal foil contact element 12, the conductive material 11 does not need to be biocompatible, and can be a solder, a metal (such as a braze), a conductive polymer or a conductive adhesive, or any other appropriate material.

Figure 17b illustrates a particular embodiment of an implantable device 8 utilising the feedthrough element 7 of figure 17. In this case, the substrate 1 may be of ceramic material, sidewall portion 5 may be of metallic material joined to substrate 1 via a metallic interlayers 3, and cover 2 may also be of metallic material, such as a metal foil. Alternatively, either one of sidewall portion 5 and cover 2 may be of ceramic material. Furthermore, if no electrical connection to the exterior is required, feedthrough element 7 may be replaced by a simple substrate 1 with no electrical feedthroughs.

Figure 18 illustrates a feedthrough element 7 similar to that illustrated in figure 17, further comprising a sidewall portion 5 attached to a base portion 1a of the substrate 1. Sidewall portion 5 may be integral with the base portion 1a of the substrate 1, or a separate part bonded thereto e.g. via a metallic interlayer 3 such as disclosed above.

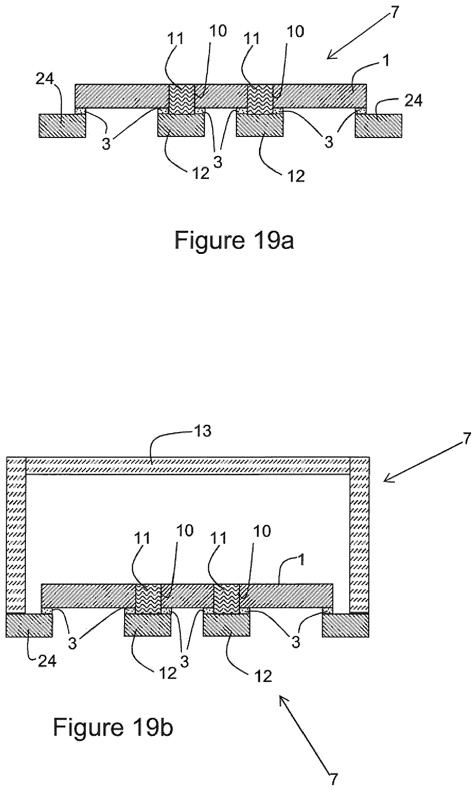

Figure 19a illustrates another feedthrough element 7 similar to that illustrated in figure 17, further comprising a peripheral metal element 24, extending around the periphery of substrate 1. Peripheral metal element 24 may be e.g. a metal foil element formed from the same material as the metal contact elements 12, and may serve for the attachment of a cover (see figure 19b) to the feedthrough element 7.

Figure 19b illustrates the feedthrough element 7 of figure 19a combined with a commercially available metal case 13 serving as cover attached to peripheral metal element 24, e.g. by laser welding, so as to form an implantable device 8. An active electronic circuit situated within the implantable device 8 is not shown in this figure. It should be noted that metal case 13 may be single-piece or multi-piece.

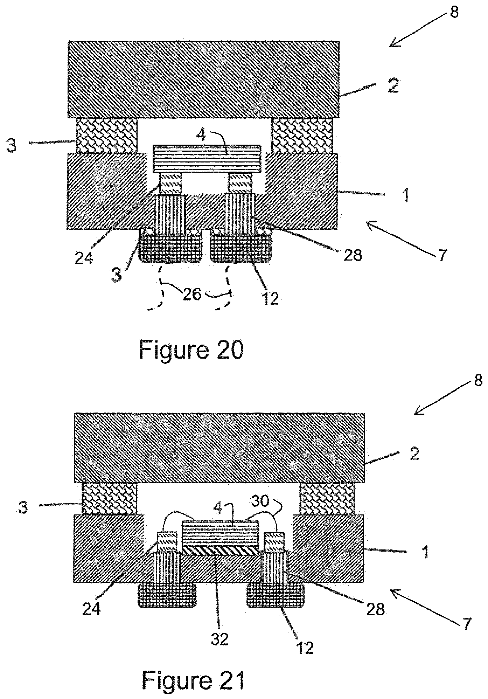

Figure 20 shows a feedthrough element 7 according to figure 18 applied to an implantable device 8. The micropackage 8 comprises a biocompatible, long-term implantable base 1, biocompatible, long-term implantable metal interlayers 3, a biocompatible, long-term implantable cover 2 and biocompatible, long-term implantable electrical interconnections 12 e.g. foils, sheet, ribbons or wires that also provide the hermetic seal of the feedthroughs. The hermetic sealing steps for the micropackage 8 were described already above, the ones for the electrical feedthroughs are presented in below. The vias 28 can have any cross-section, e.g. round or rectangular, and are filled with a conductive material such as a solder, a metal, a conductive polymer or a conductive adhesive, as described above. The active circuit 4 is bonded to the conductive via through conductive bonding pads 24 and flip-chip bonding. The conductive pad material can be any conductive material including Au. For all geometries described the via 28 filled with the conductive material can also directly act as bonding pad. An additional underfill below the active circuit can be added for increased stability (not shown in Figure 20). The electrical interconnections can also be contacted to a biocompatible long term implantable pad, wire 26 or stripe (e.g. Pt) through a standard welding or brazing process.

A similar geometry can be used for a wirebonding approach. Figure 21 shows such a feedthrough geometry for a biocompatible long-term implantable micropackage 8. The micropackage 8 comprises a biocompatible, long-term implantable base 1, biocompatible, long-term implantable metal interlayers 3 a biocompatible, long-term implantable cover 2 and biocompatible, long-term implantable electrical interconnections 12 e.g. ribbons or wires. In the most general case of electrical feedthroughs for all configurations, no metal layers 3 between base 1 and electrical interconnection 12 may be needed (e.g. by using a brazing or soldering material. The vias 28 are filled with a conductive material such as a solder, a metal or a conductive adhesive. The active circuit 4 is bonded to the conductive via through conductive bonding pads 24 and wire 30 bonding (or e.g. a combination of conductive foil bonding and laser structuring). The conductive bonding pad 24 material can be any conductive material including Au. For illustration purposes the conductive bonding pads 24 are always shown as centered on the conductive vias 28, however in general, for all geometries of flip-chip bonding and wire bonding the position of the conductive bonding pads 28 with respect to the conductive vias 28 is arbitrary as long as an electrical contact between conductive via 28 and bonding path is guaranteed. The wire bonds 30 can be embedded in a polymer for increased stability (not shown in Figure 21). As demonstrated in Figure 20 pins, ribbons or wires can be attached to the electrical interconnections 12 as well.

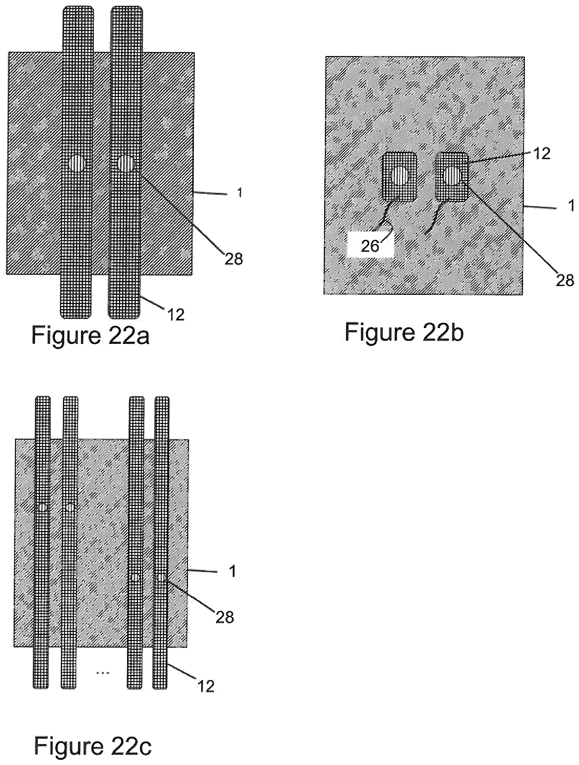

A bottom view of the approaches in Figures 20 and 21 is displayed in Figures 22a-c. The electrically conductive vias 28 are shown for clarification (depicted with a round cross-section, although other cross-sections are of course possible) and cannot in reality be seen from the bottom. The position of the vias 28 is flexible. As described below in more detail in one embodiment the electrical interconnections 12 are first bonded to the micropackage and then structured with e.g. a laser. In particular, Fig. 22a shows two electrical interconnections 12 (wires), Fig. 22b shows two electrical interconnections 12 (pads with attached pin, stripe or wires 26), and Fig. 22c shows multiple electrical interconnections 12.

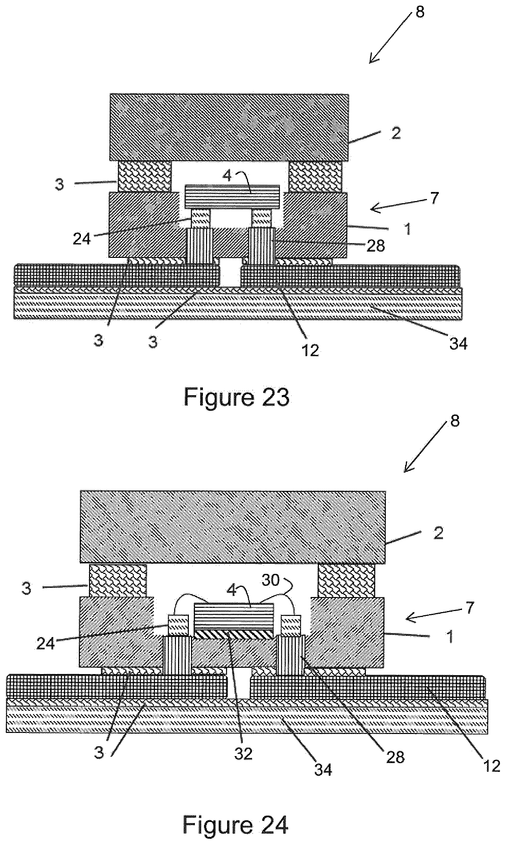

Other feedthrough geometries are discussed in the following. Figure 23 shows a feedthrough geometry for a biocompatible long-term implantable micropackage 8. The micropackage 8 comprises a biocompatible, long-term implantable base 1, biocompatible, long-term implantable metal interlayers 3, a biocompatible, long-term implantable cover 2 and electrical interconnections situated upon on further electrically nonconductive, biocompatible, long-term implantable, substrate 34 and biocompatible, long-term implantable electrical lines 12 constituting metal contact elements which also provide the hermetic sealing of the electrical feedthroughs (see above). The vias 28 are filled with a conductive material such as a solder, a metal or a conductive adhesive. The active circuit 4 is bonded to the conductive via through conductive bonding pads 24 and flip-chip bonding. As stated earlier, the metal interlayers 3 at the various interfaces can be different.

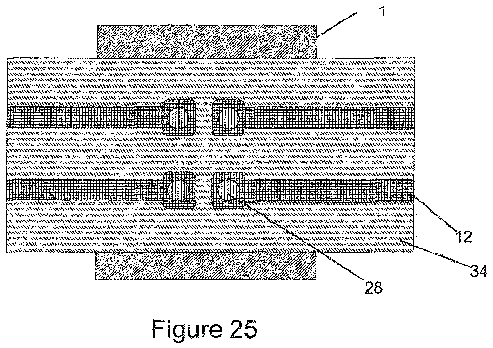

Figure 24 shows the feedthrough geometry for a biocompatible long-term implantable micropackage 8 based on wirebonding. The micropackage 8 comprises a biocompatible, long-term implantable base 1, biocompatible, long-term implantable metal interlayers 3, a biocompatible, long-term implantable cover 2 and electrical interconnections based on further electrically nonconductive, biocompatible, long-term implantable, substrate 34 and biocompatible, long-term implantable electrical lines 12. The vias 28 are filled with a conductive material such as a solder, a metal or a conductive adhesive. The active circuit 4 is bonded to the conductive via through conductive pads 24 and wire bonding 30. The bottom view of this concept is displayed in Figure 25. The electrically conductive vias 28 and electrical lines 12 are shown for clarification and cannot really be seen from the bottom. The position of the vias 28 is flexible and the electrical lines can have any shape.

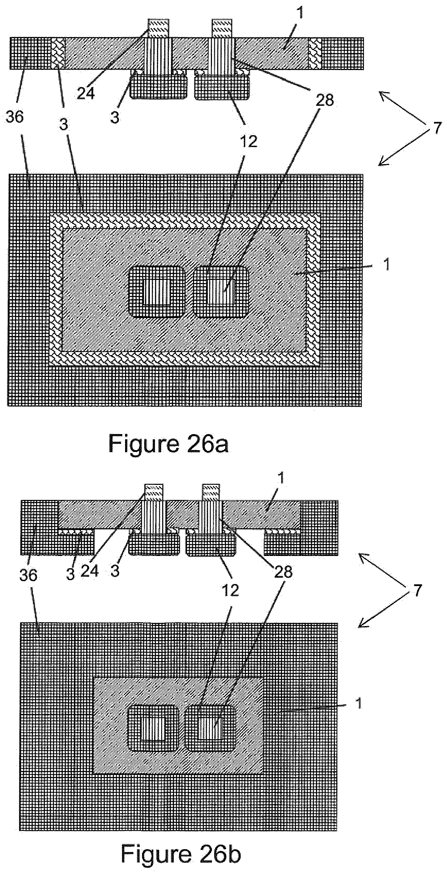

In another embodiment the feedthrough element 7 can also be stand-alone, i.e. not part of the housing. In this case an additional frame 36 may be needed to allow e.g. the welding of the feedthrough element 7 to a standard Ti housing. Figures 26a and 26b illustrate the general concept. Feedthrough substrate 1, the metal interlayers 3, the electrical interconnections 12 and the Ti flange 36 are all biocompatible and long-term implantable. In the most general case of electrical feedthroughs for all configurations in this report, no metal layers 3 between feedthrough substrate 1 and electrical interconnection 12 and/or Ti flange 36 may be needed (e.g. by using a brazing or soldering material). In a further variant, Alumina/Zirconia green tape is co-fired with electrical interconnections, e.g. foils, ribbons, or wires 12 and a flange (Ta, Pt, Nb or Ti or combinations of these materials) is hermetically sealed to the feedthrough substrate using e.g. brazing. It should be noted in all cases that the Ti flange 36 can have different shapes as is e.g. also illustrated in the patent application US 2011/000699, or any other convenient shape.

### Electrical feedthrough fabrication

In the following several methods to arrive at hermetic implantable electrical feedthrough elements 7 are described. In general, first the hermetic bonding is carried out and the vias 28 are filled afterwards. In another embodiment the sequence is exchanged. Typically, the electrical feedthroughs are hermetically sealed before the micropackage sealing. The sequence can also be exchanged. The hermetic sealing can be carried out directly with e.g. pads, wires or foils or ribbons. In another embodiment the hermetic sealing can be carried out with a metal foil and subsequently structuring (if required) into e.g. pads, wires or ribbons by e.g. laser structuring. For the laser structuring all standard and nonstandard laser structuring processes can be used. As an example the process parameters include but are not limited to: wavelength: from 250nm up to 11 microns, pulse duration: from continuous wave to fs-pulse, laser pulse repetition rate: from 0Hz to 1 MHz, laser beam area: from 10 micron to 500micron in diameter, laser beam scanning speed: from 0 to 10m/s, laser average power: 1W to 1kW. Several laser beam scanning round-trips (1-10'000) are possible. In another embodiment the laser beam is not scanned and the whole area of the structuring zone is illuminated at the same time by a suitable optical system.

### (Laser-assisted) diffusion bonding

Diffusion bonding has already been described above and will therefore not be described again.

For electrical interconnections, a bulk material such as a foil or a wire which has much lower yield strength compared to thin films is bonded to a biocompatible substrate, e.g. alumina or sapphire, with metal interlayers. This results in a low force plastic deformation due to the low yield strength of the bulk material and low temperature bond formation due to grain boundaries and/or the high dislocation density of thin films.

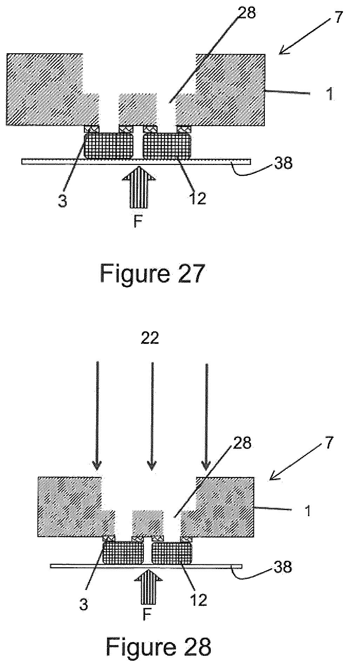

Figure 27 shows diffusion bonding for the hermetic sealing of electrical interconnections to a feedthrough element 7 for an implantable micropackage. The feedthrough element comprises a biocompatible, long-term implantable base 1. The hermetic sealing of the electrical contact is provided through biocompatible, long-term implantable metal interlayers 3 diffusion bonded to electrical interconnections 12. A normal force F and temperature is applied to a (removable) plate 38 during the bonding process. The active circuits (not shown) are connected to the electrical interconnections with vias 28 (description see below). For clarity, the micropackage cover is not shown. The process parameters may be in the following range: temperature: from 400 to 1600 degrees, process time: from few seconds to 30 mins, pressure: from 5-150MPa.

Laser assisted diffusion bonding can also be used for the hermetic sealing of electrical interconnections to a feedthrough element 7 of an implantable micropackage (Figure 28). The feedthrough element 7 comprises a biocompatible, long-term implantable base 1. The hermetic sealing of the electrical contact is provided through biocompatible, long-term implantable metal interlayers 3 laser assisted diffusion bonded to electrical interconnections 12. A normal force (and temperature) F is applied to a (removable) plate 38 during the bonding process. The active circuits (not shown) are connected to the electrical interconnections through vias 28. For clarity, the micropackage cover is not shown. The process parameters may be in the following range: wavelength: from 250nm up to 11 microns, pulse duration: from continuous wave to fs-pulse (various pulse shapes), laser pulse repetition rate: from 0Hz to 1 MHz, laser beam area: from 10 micron to 500micron or even to 1000micron in diameter, laser beam scanning speed: from 0 to 10m/s, laser average power: 1W to 1kW, or even to 10kW. Several laser beam scanning round-trips (1 -10'000) are possible. In another embodiment the laser beam 22 is not scanned and the whole area of the welding zone is illuminated at the same time by a suitable optical system. Normal forces are in the range of 5 to 20 MPa and bonding times are in the ms to s range.

If the (removable) plate 38 for bonding is transparent for the laser wavelength used, the laser beam 22 can also be incident from that side. In another embodiment the laser beam can also be incident from both sides.

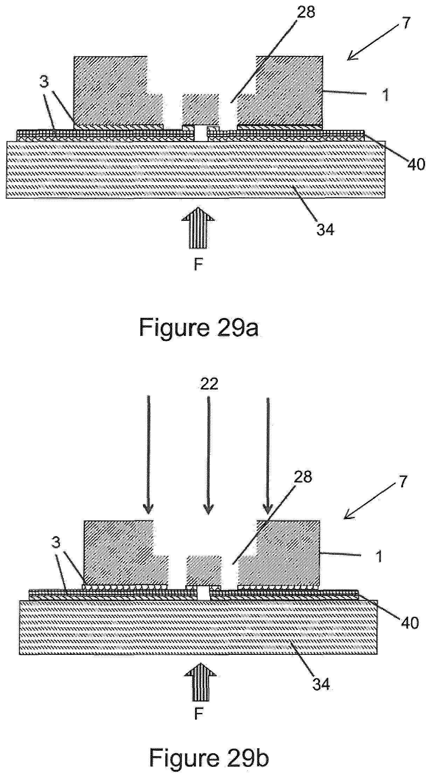

Figure 29 shows another embodiment of diffusion bonding for the hermetic sealing of electrical interconnections to a feedthrough element 7 for an implantable micropackage. The feedthrough element 7 comprises a biocompatible, long-term implantable base 1 with metal interlayers 3. The hermetic sealing of the electrical contact is provided through biocompatible, long-term implantable thin metal film 40 diffusion bonded to a further electrically nonconductive, biocompatible, long-term implantable, substrate 34 with metal interlayers 3. A normal force and temperature F is applied during the bonding process. The active circuits (not shown) are connected to the electrical interconnections through vias 28. For clarity, the micropackage cover is not shown. The (structured) thin metal film 40 acting e.g. as electrical lines can e.g. be connected with biocompatible wires, ribbons or pins for further electrical interconnectivity, see above (not shown in Figure 29).

Figure 29b shows the same configuration as in Figure 29a for laser-assisted diffusion bonding. The feedthrough element 7 comprises a biocompatible, long-term implantable base 1 that is transparent for the laser 22 wavelength and has metallic interlayers 3 on the bonding side. The hermetic sealing of the electrical contact is provided through a biocompatible, long-term implantable thin metal film 40 laser assisted diffusion bonded to a further electrically nonconductive, biocompatible, long-term implantable, substrate 34 with metal interlayers 3. A normal force (and temperature) F is applied during the bonding process. The active circuits (not shown) are connected to the electrical wires with feedthroughs. For clarity, the micropackage cover is not shown.

In a similar way as described for Figure 29, the laser beam can also be incident from the substrate side if the substrate is transparent or from both sides.

In all cases, the vias can be formed through a variety of materials including isotropic conductive adhesives, anisotropic conductive adhesives, and metals and a variety of deposition techniques including dispensing, jetting, screen printing, galvanic deposition, and sputtering.

For the stand-alone feedthroughs described above, the Ti flange can be hermetically sealed to the feedthrough substrate e.g. by using known methods such as those described in the patent application US 2011/000699 A1).

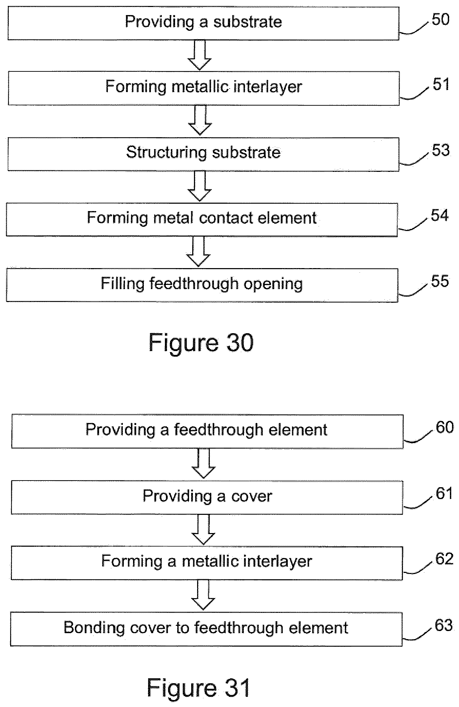

Figure 30 shows most generically a method of manufacturing a feedthrough element according to the present description.

In step 50, a substrate is provided. The substrate would typically be constructed of biocompatible ceramic. Subsequently, the metallic interlayer as described above is formed upon the substrate in step 51, and in step 53, the substrate is structured so as to form the feedthrough openings, and also to give the substrate its final shape if applicable. In step 54, the metal contact element is formed, e.g. either by bonding a wire or strip to the metallic interlayer, or by bonding a metal foil to the metallic interlayer, the metal foil being subsequently structured. This bonding is carried out by at least one of diffusion bonding, laser assisted diffusion bonding, laser welding, thermosonic welding, and ultrasonic welding. Finally, in step 55, the feedthrough opening is filled. Alternatively, steps 54 and 55 may be reversed.

Figure 31 shows most generically a method of manufacturing an implantable device according to the description. In step 60 a biocompatible feedthrough element is provided, which may be of a type described above, or may be any other convenient type, and in step 61 a biocompatible cover is provided. In step 62, a metal interlayer is formed on one or both of the feedthrough element and/or the cover. Naturally, the feedthrough element and/or the cover may already be provided with the metallic interlayer. Subsequently, the cover is bonded to the feedthrough element by at least one of diffusion bonding, laser assisted diffusion bonding, laser welding, thermosonic welding, and ultrasonic welding. Naturally, the feedthrough element may comprise an intermediate wall element to which the cover is bonded, the intermediate wall element being provided as a separate part and subsequently bonded to the substrate of the feedthrough element before or after the cover is bonded thereto.

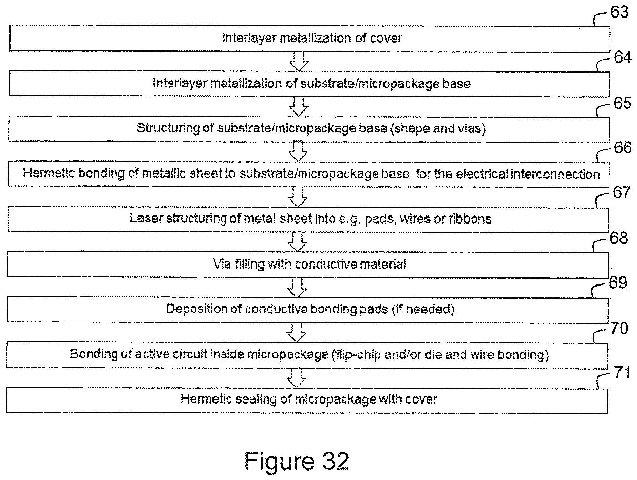

Figure 32 illustrates a more detailed method of manufacturing an implantable device according to the description. In step 70, a previously-provided cover is metallised to form a metallic interlayer, and in step 64 a metallic interlayer is likewise formed on the substrate of the feedthrough element, which will form the base of the implantable device/micropackage. In step 65, the substrate is structured so as to form at least one feedthrough opening for vias and, if applicable, to give the substrate its final shape. In step 66, a metallic sheet is hermetically bonded to the substrate, and is then laser structured in step 67 so as to form pads, wires, ribbons etc. forming metal contact elements. In step 68, the feedthrough opening(s) are filled with a conductive material such as a solder, a braze, or a conductive polymer or adhesive so as to form one or more vias. In step 69, if appropriate, conducted bonding pads are deposited. In step 70, an active circuit is bonded to the feedthrough element such that it will end up inside the micro package, by flip-chip and/or die and wire bonding. Finally, in step 71, the implantable device is hermetically sealed with its cover, which is bonded by at least one of diffusion bonding, laser assisted diffusion bonding, laser welding, thermosonic welding, and ultrasonic welding.

### Electrical interconnections

In all cases the electrical interconnection between the micropackages and to other electrical components can be carried out using established methods including welding and brazing of e.g. Pt wires and pins.

Electrical interconnections between the micropackages can also be realized through a chip on wire approach which can be important e.g. for an array of active implants. In one embodiment of such an approach gaps or grooves are required to fix a wire in between. Such a gap can e.g. be formed with a cap fixed to a substrate or package.

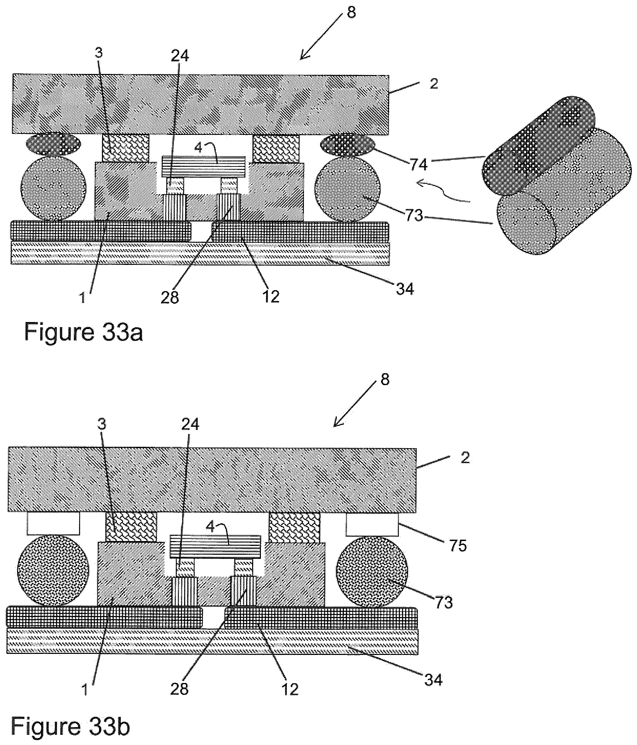

However, the electrical interconnections between the chips can also be made using a biocompatible elastomeric/ plastic wire 73 which is wound using a metal wire. This wire 73 is press fitted into a groove between the cover 2 and substrate 1 which can be formed using a spacer. The thickness of the wire 73 is chosen in such a way that compression of the elastic wire or slight plastic deformation of plastic wire will make the electrical connection to the pads 12 on the active substrate as shown in Figure 33a. The comprises a biocompatible, long-term implantable base 1, biocompatible, long-term implantable metal interlayers 3 (not all interlayers are shown), a biocompatible, long-term implantable cover 2 and electrical interconnections based on further electrically nonconductive substrate 34 which is biocompatible and long-term implantable, and biocompatible, long-term implantable electrical lines 12. The hermetic sealing steps, the vias and the active circuit bonding were described already above. The electrical interconnection is built with a (deformable) electrical conductive biocompatible long-term implantable wire 73 and a deformable elastic biocompatible long-term implantable wire 74. For clarity the metal interlayers between bottom of housing 1 and electrical lines 12 as well as between the electrical lines 12 and the further electrically nonconductive, biocompatible, long-term implantable, substrate 34 are not shown. When inserted into the gap between bottom of housing and cap the elastic wire 74 is deformed holding the conductive wire 73 in place. This bond can be further stabilized by the application of a biocompatible long-term implantable adhesive such as Silicone. The metal and elastic wires can be attached to each other, e.g. by braiding or knitting. The concept can be extended to several electrical feedthroughs in a micropackage using insulated wires with conductive openings at positions where the electrical interconnections have to be or using wires with e.g. Pt based isotropic and/or anisotropic conductive adhesives. In another embodiment a fixation with a medically approved isotropic and/or anisotropic adhesive can be applied on metal wires or metal wires wound on an elastomeric/plastic wire.

Fig.33b shows another embodiment in which the electrical interconnection is built with a (deformable) electrical conductive biocompatible long-term implantable wire 73 and a deformable elastic biocompatible long-term implantable bump or layer 75 already attached to cover 2 or further electrically nonconductive, biocompatible, long-term implantable, substrate 34. When inserted into the gap between bottom of housing and cap the elastic bump 75 is deformed holding the conductive wire 73 in place. This bond can be further stabilized by the application of a biocompatible long-term implantable adhesive.

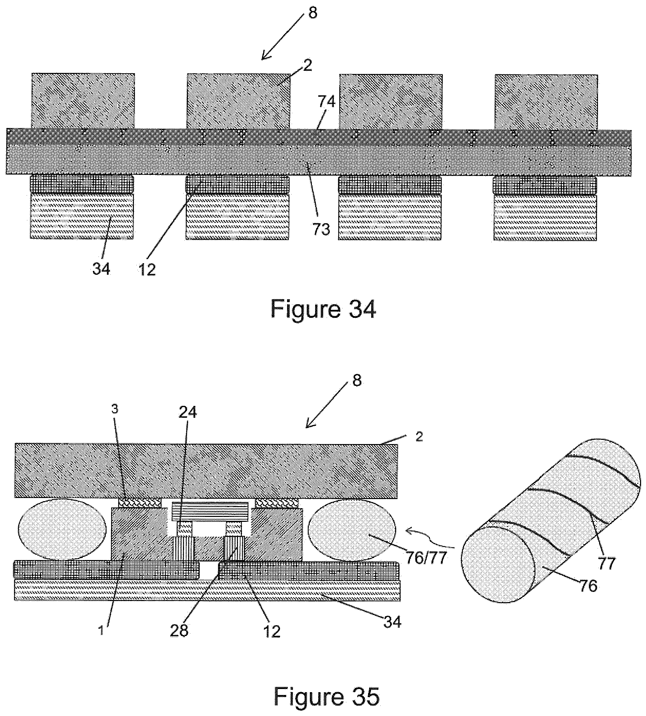

In such a way, an array of active implants can be connected in series as shown in side view in Figure 34. A stretchable wound elastomer/plastic wire 73, 74 is attached between cap or cover 2 and further electrically nonconductive, biocompatible, long-term implantable, substrate 34 electrical lines 12 as described above and below. For clarity, all metal interlayers are omitted in this figure.

Another embodiment for electrical interconnections is shown in Figure 35 which shows an electrical interconnection to a biocompatible long-term implantable device 8. The micropackage 8 consists of a biocompatible, long-term implantable base 1, biocompatible, long-term implantable metal interlayers 3 (not all shown for clarity), a biocompatible, long-term implantable cover 2 and electrical interconnections based on further electrically nonconductive, biocompatible, long-term implantable, substrate 34 and biocompatible, long-term implantable electrical lines 12. The hermetic sealing steps, the vias 28 and the active circuit bonding have already been described above. The electrical interconnection is built with an electrical conductive biocompatible long-term implantable wire 76 connected (e.g. winding, weaving,...) to a deformable elastic biocompatible long-term implantable metal wire 77. For clarity the metal interlayers between bottom of housing 1 (i.e. the substrate of the feedthrough element) and electrical lines 12 as well as between the electrical lines 12 and the further electrically nonconductive, biocompatible, long-term implantable, substrate 34 are not shown. The elastic wire is stretched to insert it into the gap between bottom of housing and cap 2. When releasing the wire it is held in place by the tension of the elastic wire 76. This bond can be further stabilized by the application of a biocompatible long-term implantable adhesive. The concept can be extended to several electrical feedthroughs in a micropackage using insulated wires with conductive openings at positions where the electrical interconnections have to be or using wires with e.g. Pt based isotropic and/or anisotropic conductive adhesives. In another embodiment a fixation with a medically-approved isotropic and/or anisotropic adhesive can be applied on metal wires or metal wires wound on an elastomeric/plastic wire.

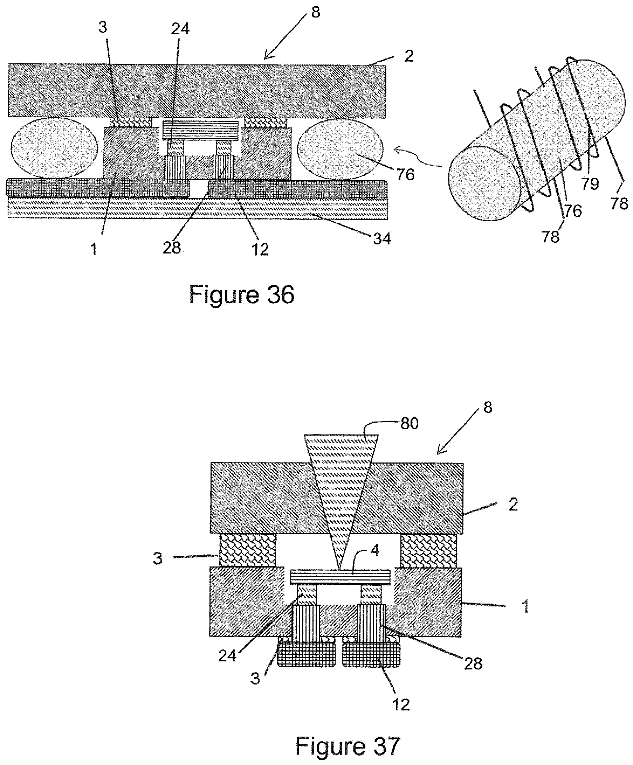

Another embodiment for electrical interconnections is shown in Figure 36 which shows multiple electrical interconnections to a biocompatible long-term implantable micropackage 8. The micropackage 8 comprises a biocompatible, long-term implantable base 1, biocompatible, long-term implantable metal interlayers 3, a biocompatible, long-term implantable cover 2 and biocompatible, long-term implantable electrical interconnections based on an electrically non-conductive substrate 34 and electrical lines 12. The hermetic sealing steps, the vias and the active circuit bonding were described already above. The electrical interconnection is built with an electrically conductive biocompatible long-term implantable wire 76 connected (e.g. wound,...) with a deformable elastic biocompatible long-term implantable wire 79. For clarity the metal interlayers between bottom of housing 1 and electrical lines 12 as well as between the electrical lines 12 and the further electrically nonconductive, biocompatible, long-term implantable, substrate 34 are not shown. Isolated wire endings cut to bond multiple wires, coming from for example passive electrodes 78. The elastic wire is stretched to insert it into the gap between bottom of housing and cap 2. When releasing the wire it is held in place by the tension of the elastic wire. The wire endings are then cut to be accessible for the connection of additional wires. These bonds can be further stabilized by the application of a biocompatible long-term implantable adhesive.

### Implantable light sources and vision sensors

For a number of applications including optical neurostimulation, glucose monitoring, UV sterilization and optical imaging, active photonic components need to be implanted for a long time. For this, the methods described above can be utilized and several concepts based on them are described in the following. In the examples below the active photonics components are attached through flip-chip bonding. It is clear that die attachment and wire bonding are other means that can be used to integrate the active photonics components into the micropackage.

Figure 37 shows a geometry for a biocompatible long-term implantable micropackage 8 for a light source 4. Light source means a single source, multiple sources with or without associated electronics. The light emitting element can be any light emitting element including LEDs, VCSEL's and lasers. The micropackage 8 comprises a biocompatible, long-term implantable base 1, biocompatible, long-term implantable metal interlayers 3, a biocompatible, long-term implantable, for the emitted light beam transparent cover 2 and biocompatible, long-term implantable electrical interconnections 12 e.g. wires. The vias 28 are filled with a conductive material such as a solder, a metal or a conductive adhesive. The light source 4 is bonded to the electrical vias 28 through conductive bonding pads 24 and flip-chip bonding. As mentioned before, for all geometries described the via 28 filled with the conductive material can also directly act as bonding pad. The light 80 is emitted through the transparent cover 2 (e.g. Sapphire)

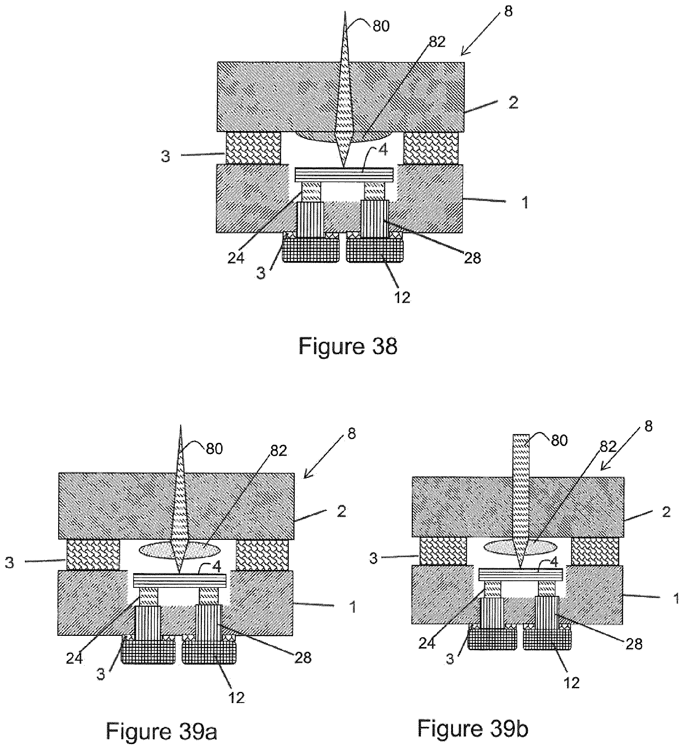

Figure 38 shows another embodiment for a biocompatible long-term implantable micropackage 8 for a light source. The micropackage 8 comprises a biocompatible, long-term implantable base 1, biocompatible, long-term implantable metal interlayers 3, a biocompatible, long-term implantable cover 2, transparent for the emitted light beam, with integrated lens 82 and biocompatible, long-term implantable electrical interconnections 12 e.g. wires. The vias 28 are filled with a conductive material such as a solder, a metal or a conductive adhesive. The light source 4 is bonded to the electrical vias 28 through conductive bonding pads 24 and flip-chip bonding. The light is emitted through the transparent cover 2. The lens 82 which can be part of the housing cap/cover 2 (either as one piece or fixed to the cap 2 (e.g. with an adhesive)) modifies the beam profile 80 as required for the intended application. The lens 82 can be convex, biconvex, concave or biconcave depending on the application. The lens 82 can also be an array of microlenses. In another embodiment, the can also be mounted outside the package (not shown in Fig.38).

In another embodiment, the lens 82 can be integrated in the package and not in the cover 2. The lens 82 may also be tuneable to allow a modification of the beam 80 such as focusing or defocusing. Figures 39a (focused beam) and 39b (parallel beam) show a geometry for a biocompatible long-term implantable micropackage 8 for a light source 4. The micropackage 8 consists of a biocompatible, long-term implantable base 1, biocompatible, long-term implantable metal interlayers 3 a biocompatible, long-term implantable cover 2, transparent for the emitted light beam, and biocompatible, long-term implantable electrical interconnections 12 e.g. wires. The vias 28 are filled with a conductive material such as a solder, a metal or a conductive adhesive. The light source 4 is bonded to the electrical interconnections via through conductive bonding pads 24 and flip-chip bonding. The light is emitted through the transparent cover 2. The tuneable lens 82 modifies the beam profile 80 as required for the intended application. For clarity the electrical connections and the fixation of the tuneable lens 82 are not shown.

In a further embodiment, an optical grating either fixed or electrically tuneable can be part of the cover/cap 2 or the micropackage 8 to extend the optical functions.

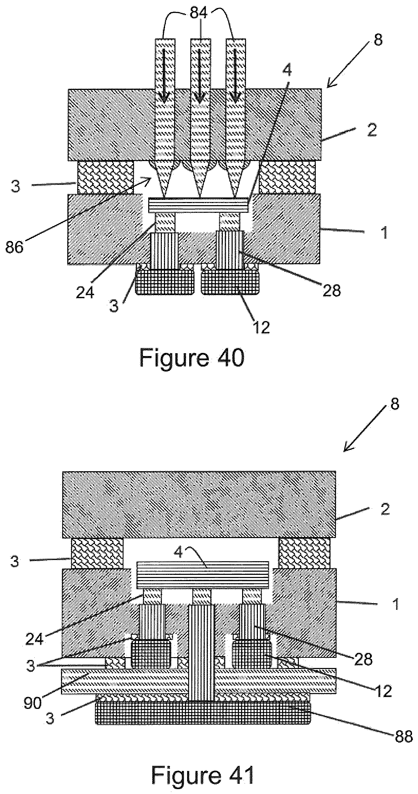

Based on the same concept, vision sensors can be integrated into a longterm implantable micropackage 8. Vision sensor means one or several CCD or CMOS chips and/or one or several photodiode with or without associated electronics. In this case the light is passing from the outside through the cap (transparent for the wavelength used) and incident on the vision sensor (Figure 40). Again, optical functions including single lenses 82 (convex, concave, biconvex, biconcave), microlens arrays 86, and gratings can be incorporated either directly into the cap 2 or into the micropackage 8.

In addition, combinations of light sources and vision sensors in a single package or in multiple packages can be realized for applications involving optical stimulation and optical measurement including e.g. light based sensors such as e.g. glucose monitoring or long term endoscopy.

### Implantable intelligent electrodes

In applications such as electrical neurostimulation (including spinal cord, sacral nerve, deep brain, cochlear) stimulation and recording from a large number of electrodes is feasible if each electrode is made intelligent by the integration of active circuits into a micropackage. Figure 41 shows a feedthrough geometry for a biocompatible long-term implantable micropackage 8. The micropackage 8 comprises a biocompatible, long-term implantable base 1, biocompatible, long-term implantable metal interlayers 3, a biocompatible, long-term implantable cover 2 and dielectric layer 90, biocompatible, long-term implantable electrical interconnections 12 e.g. wires, and a biocompatible, long-term implantable electrical electrode 88. The vias 28 are filled with a conductive material such as a solder, a metal or a conductive adhesive. The active circuit 4 is bonded to the electrical interconnections 12 and electrodes 88 via through conductive bonding pads 24 and flip-chip bonding (or die attach and wire-bonding (not shown in Figure 41)).

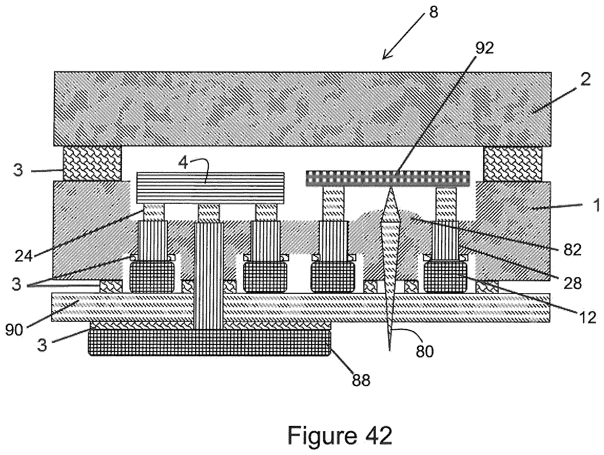

Simultaneous optical stimulation and electrical recording can be realized either by the use of separate micropackages or by one highly integrated micropackage 8. Figure 42 shows a feedthrough geometry for a biocompatible long-term implantable micropackage 8. The micropackage 8 comprises a biocompatible, long-term implantable base 1, biocompatible, long-term implantable metal interlayers 3, a biocompatible, long-term implantable cover 2 and dielectric layer 90 (transparent for the wavelengths used), biocompatible, long-term implantable electrical interconnections 12 e.g. wires, and a biocompatible, long-term implantable electrical electrode 88. The vias 28 are filled with a conductive material such as a solder, a metal or a conductive adhesive. The active circuit 4 is bonded to the electrical interconnections and electrodes via through conductive pads 24 and flip-chip bonding. The light source 92 is bonded to the electrical interconnections 12 via through conductive bonding pads 24 and flip-chip bonding. The light 80 is emitted through the transparent bottom of the housing of the micropackage 8. The lens 82, which can be part of the housing cap or can be integrated into the micropackage 8, modifies the beam profile as required for the intended application. In another embodiment of the description the light beam 80 can point in a direction different from the electrode surface normal. As an example, the light can exit through the cap 2 of the housing. In this case the cap 2 needs to be transparent for the wavelengths used and may contain optical elements (lenses, gratings,...) as described before. As a further embodiment the optical function can be extended through the replacement of the lens 82 with an optical grating or through a combination of lenses and optical gratings.

### Optical neurostimulation

The most widely-used current method of artificial stimulation of neural tissue for pain relief, therapeutic processes and sensory recovery, e.g. of auditory functions, is electrical. Because of electrode-tissue interface this technique has many limitations including damage to neural tissue by high current or mechanical contact, susceptibility to environmental interference, and introduction of high-frequency artefacts to the stimulation signal. The performance of electrical stimulation of the sensory system as e.g. the hearing is limited by the extent to which the electric field can be controlled either by position of electrode, which may require penetrating the neural tissue, or field shaping by controlling the delivered current. It is unlikely that substantial advances will be made in improving the resolution of existing neuromodulation systems without changing the means for generation of action potentials. Optical neurostimulation offers significantly increased spatial and temporal selectivity when compared with electrical stimulation and to overcome the drawbacks of electrical stimulation.

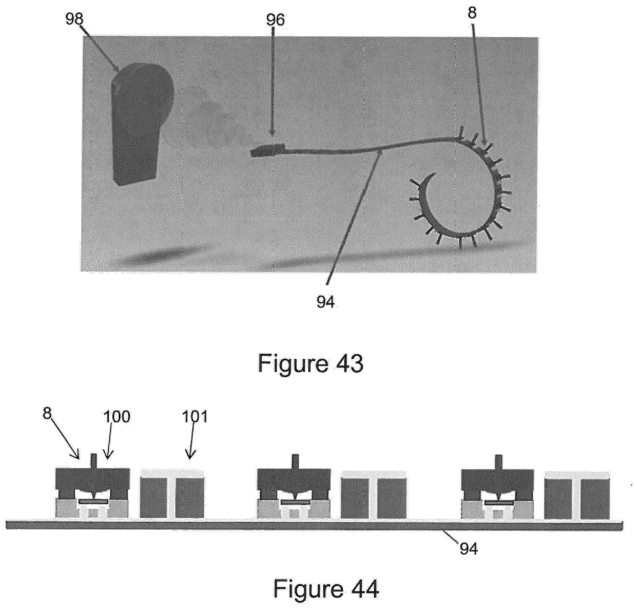

The state of the art of optical neurostimulation uses optical fibers. Light coupled is coupled into the fiber(s) and guided to the location of stimulation where several mechanisms are used to extract the light. Such approaches are useful for first tests but are by no means suited for actual products as they are too bulky and complicated (e.g. it is difficult to address different stimulation locations independently). Here is proposed a different approach in which individual light sources, e.g. VCSEL's are packaged in one or two-dimensional arrays using the technologies described above. In such configurations each light source can be individually addressed. Figs. 43 and 44 show configurations with both optical stimulation and electrical recording (see below). Obviously, all combinations with and without electrical recording are possible and the light sources can be arranged in different ways (along a line, as shown in figs 43 and 44 or in a two-dimensional array.

There are several significant challenges towards the realization of a system based on optical neurostimulation: power consumption, size and heat generation of the light source and packaging of the system in a way, which keeps the integrated system biocompatible. Furthermore, the acquisition of neural response from stimulating electrodes with highest sensitivity is required as an evoked response for identifying the extent and nature of the depolarisations produced by optical means and an improvement of the sensory recovery.

With the implantable micropackage solutions presented here, optical stimulation and/or electrical stimulation and/or electrical recording can be realized in a highly integrated fashion for applications including spinal cord stimulation, sacral nerve stimulation, deep brain stimulation and cochlear implants. Figures 43 and 44 show the example of a cochlear implant with optical stimulation and electrical recording. In this case the individual implantable micropackages 8 should have a size down to below 1 mm x 1 mm 1 mm. These implantable micropackages 8 are distributed along a so-called flexprint 94, and are in communication with an implantable neurostimulation and recording module (INSTAR) 96. INSTAR 96 communicates wirelessly with an external energy transfer and communication module 98 (EXTRACOM). Figure 40 shows in detail in cross-section an arrangement of packaged light sources 100 comprising a laser for optical stimulation, constructed as a micropackage 8. Adjacent to each light source 100 is an evoked response electrode 102. Both the light sources 100 and the evoked response electrodes 102 are formed on flexprint 94, and are electrically interconnected. For other applications such as e.g. optical spinal cord stimulation or optical deep brain stimulation, other geometries and sizes can be envisioned and a number of features can be adapted including the number of light sources and electrical recording electrodes (the number of light sources and recording electrodes can be different), the arrangement and position of these components (in a line or in an array), the flexprint material, and more.

The flexprint material selection includes biocompatible polyurethane and silicone elastomers and duromers. Metal line materials to guide all electrical signals in the flex include platinum, titanium, alumina, sapphire, zirconia, tantalum, nitinol, niobium and some cobalt chromium alloys. In addition, although not yet proven to be long-term implantable materials such as gold, silver and palladium are considered as well.

In addition to the optical stimulation and electrical recording units also the INSTAR module as illustrated in Fig.43 can be packaged with the technologies described above.

The electric connection between the optical stimulation and/or electrical recording unit to the flex can be carried out using standard bonding processes including soldering, thermocompression, adhesive fixing (isotropic conductive, nonconductive, anisotropic conductive adhesives).

### Optimisation for wireless applications

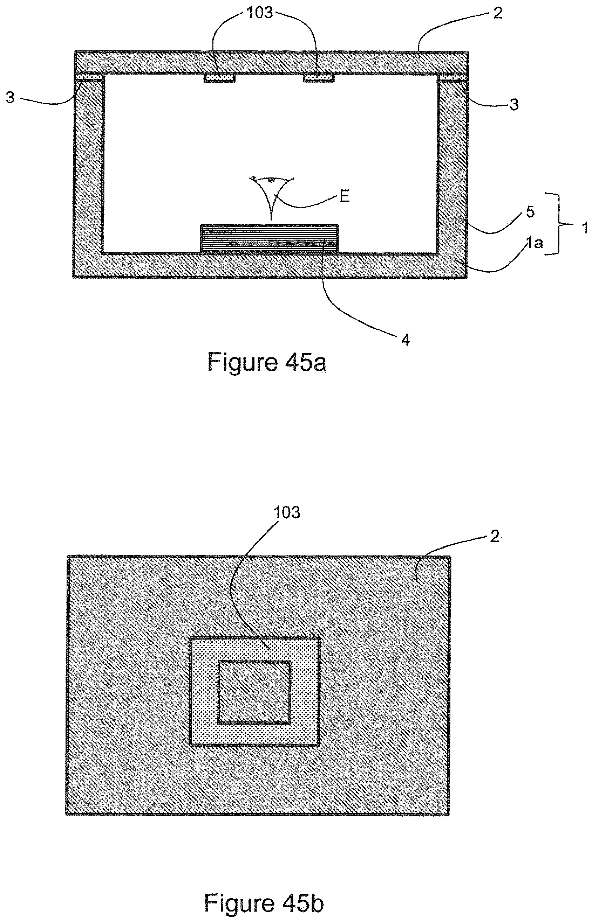

Finally, figures 45a and 45b illustrate an implantable device 8 optimised for wireless applications, i.e. applications in which electronic device 4 is powered and communicates wirelessly. The implantable device comprises a hollow base 1, which in this example comprises a substrate 1a and an integral sidewall 5, in the interior of which is situated an electronic device 4. Cover 2 is bonded to sidewall 5 via a metallic interlayer 3 as described above. Cover 2, substrate 1 a and sidewall 5 are of a dielectric material such as a ceramic. Furthermore, cover 2 comprises on its interior side an antenna 103 formed of a ring of metallic material, e.g. the same material as the metallic interlayers 3, which can be formed at the same time as the metallic interlayers 3. Alternatively, antenna 103 may be formed by a metal foil, a deposited metal layer of a different metallic material, or similar.

As illustrated in figure 45b, which represents a view of the interior side of cover 2 of implantable device 8, viewed from the perspective of the eye symbol E on figure 45a, antenna 103 is formed as a square ring. However, other forms are possible such as circular, oval, or other shapes. Antenna 103 may be in electrical connection with electronic device 4, or may simply serve so as to focus radio waves onto a further antenna forming part of electronic device 4, thereby improving wireless reception. Furthermore, antenna 103 may simultaneously serve to receive wireless power as a remote power antenna. Antenna 103 may have, but is not limited to, the following dimensions: diameter 500µm - 3mm; line width 75µm -300µm.

Although the implantable devices described herein are primarily intended to be implanted in the body, it should be noted that they are also suitable to be used in harsh environments.

The invention is set out in the following claims:

## Claims

1. Implantable device (8) comprising:
- a base element (1; 7);
- a cover (2) attached to said base element (1; 7) so as to define a cavity arranged to receive an electronic device (4);
- a metallic interlayer (3) disposed between the base element (1) and the cover (2), and arranged so as to bond the base element (1; 7) to the cover (2), wherein said metallic interlayer (3) comprises at least one bonding layer (3b), charachterised in that said metallic interlayer (3) further comprises at least one diffusion barrier layer (3a).

2. Implantable device (8) according to claim 1, wherein said cover (2) is substantially transparent.

3. Implantable device (8) according to claim 2, further comprising at least one lens (82; 86) integral with, or attached to, said cover (2).

4. Implantable device (8) according to one of claims 1-3, wherein said metallic interlayer (3) further comprises at least one absorption layer (3c) adapted to absorb laser light, said absorption layer (3c) prefereably constituting an adhesion layer.

5. Implantable device (8) according to claim 4, wherein said metallic interlayer (3) comprises the sequence of layers titanium - tantalum - platinum, preferably the sequence of layers titanium - tantalum - platinum - tantalum - titanium.

6. Implantable device (8) according to any of claims 1-5, wherein said implantable device is a MEMS microphone or a MEMS pressure sensor.

7. Method of manufacturing an implantable device (8) according to any of claims 1-6, comprising the steps of:
- providing a base element (1; 7) comprising a substrate (1);
- providing a cover (2);
- forming a metallic interlayer (3) on at least one of said base element and said cover, said metallic interlayer (3) comprising at least one diffusion barrier layer (3a) and at least one bonding layer (3b);
- bonding the cover (2) to the base element (1; 7) by means of at least one of:
- diffusion bonding;
- laser assisted diffusion bonding;
- laser welding;
- thermosonic welding;
- ultrasonic welding.

8. Feedthrough element (7) for an implantable device (8) comprising :
- a substrate (1) comprising at least one feedthrough opening (10), said feedthrough opening (10) being hermetically closed on a first side of said substrate (1) by a metal contact element (12) having a width greater than the width of the repective feedthrough opening (10), and said feedthrough opening being filled with an electrically conductive material (11);
- a metallic interlayer (3) bonding said metal contact element (12) to said substrate (1) around the circumference of said feedthrough opening (10), said metallic interlayer (10) comprising at least one bonding layer (3b), **characterised in that** said metallic interlayer (10) further comprises at least one diffusion barrier layer (3a).

9. Feedthrough element (7) according to the preceding claim, wherein the metal contact element (12) is a metal foil contact element.

10. Feedthrough element (7) according to claim 8 or 9, further comprising :
- a peripheral metal element (24), preferably a metal foil element, protruding from the periphery of the substrate (1), preferably arranged on said first side of said substrate (1);
- a further metallic interlayer (3) bonding the peripheral metal element (24) to said substrate (1).

11. Feedthrough element (7) according to any of claims 8-10, wherein said metal contact element (12) constitutes at least one of: an electrical pad; an electrical track.

12. Feedthrough element (7) according to any any of claims 8-11, further comprising a flange (36) attached to the periphery of the substrate (1).

13. Feedthrough element (7) according to any any of claims 8-12, wherein said metallic interlayer (3) further comprises at least one absorption layer (3c) adapted to absorb laser light.

14. Feedthrough element (7) according to claim 13, wherein said metallic interlayer (3) comprises the sequence of layers titanium - tantalum - platinum, preferably the sequence of layers titanium - tantalum - platinum - tantalum - titanium.

15. Implantable device (8) comprising :
- a feedthrough element (7) according to any of claims 8-14;
- a cover (2) attached directly or indirectly to said feedthrough element (7) so as to define a hermetically sealed cavity;
- an electronic device (4) disposed in said cavity in electrical connection with said metal contact element (12).

16. Implantable device (8) according to claim 15, wherein wherein said implantable device is a MEMS microphone or a MEMS pressure sensor.

17. Method of manufacturing a feedthrough element (7) according to one of claims 8-14, comprising the steps of:
- providing a substrate (1);
- forming at least one metallic interlayer (3) upon at least part of said substrate (1), said metallic interlayer (3) comprising at least one diffusion barrier layer (3a) and at least one bonding layer (3b);
- structuring said substrate (3) so as to form at least one feedthrough opening (10);
- forming a metal contact element (12) so as to close said feedthrough opening (10), said metal contact element (12) having a width greater than the width of said feedthrough opening (10);
- filling said feedthrough opening (10) with an electrically conductive material (11),
wherein said metal contact element (12) is bonded to the substrate (1) by means of said metallic interlayer (3), said bonding being carried out by at least one of:
- diffusion bonding;
- laser assisted diffusion bonding;
- laser welding;
- thermosonic welding;
- ultrasonic welding.

## Patentansprüche

1. Implantierbare Vorrichtung (8), die umfasst:
- ein Basiselement (1; 7);
- eine Abdeckung (2), die an dem Basiselement (1; 7) angebracht ist, um einen Hohlraum zu definieren, der so ausgelegt ist, dass er eine elektronische Vorrichtung (4) aufnimmt;
- eine metallische Zwischenschicht (3), die zwischen dem Basiselement (1) und der Abdeckung (2) angeordnet und so ausgelegt ist, dass sie das Basiselement (1; 7) an die Abdeckung (2) bondet, wobei die metallische Zwischenschicht (3) zumindest eine Bondingschicht (3b) umfasst,
**dadurch gekennzeichnet, dass** die metallische Zwischenschicht (3) des Weiteren zumindest eine Diffusionsbarriereschicht (3a) umfasst.

2. Implantierbare Vorrichtung (8) nach Anspruch 1, wobei die Abdeckung (2) im Wesentlichen transparent ist.

3. Implantierbare Vorrichtung (8) nach Anspruch 2, die des Weiteren zumindest eine Linse (82; 86) umfasst, die mit der Abdeckung (2) integral ausgebildet oder daran angebracht ist.

4. Implantierbare Vorrichtung (8) nach einem der Ansprüche 1 bis 3, wobei die metallische Zwischenschicht (3) des Weiteren zumindest eine Absorptionsschicht (3c) umfasst, die so ausgelegt ist, dass sie Laserlicht absorbiert, wobei die Absorptionsschicht (3c) vorzugsweise eine Adhäsionsschicht darstellt.

5. Implantierbare Vorrichtung (8) nach Anspruch 4, wobei die metallische Zwischenschicht (3) die Abfolge von Schichten Titan - Tantal - Platin umfasst, vorzugsweise die Abfolge von Schichten Titan - Tantal - Platin - Tantal - Titan.

6. Implantierbare Vorrichtung (8) nach einem der Ansprüche 1 bis 5, wobei die implantierbare Vorrichtung ein MEMS-Mikrophon oder ein MEMS-Drucksensor ist.

7. Verfahren zum Herstellen einer implantierbaren Vorrichtung (8) nach einem der Ansprüche 1 bis 6, das die Schritte umfasst:
- Bereitstellen eines Basiselements (1; 7), das ein Substrat (1) umfasst;
- Bereitstellen einer Abdeckung (2);
- Bilden einer metallischen Zwischenschicht (3) auf zumindest einem des Basiselements und der Abdeckung, wobei die metallische Zwischenschicht (3) zumindest eine Diffusionsbarriereschicht (3a) und zumindest eine Bondingschicht (3b) umfasst;
- Bonden der Abdeckung (2) an das Basiselement (1; 7) mithilfe zumindest einem von:
- Diffusionsbonding;
- lasergestütztem Diffusionsbonding;
- Laserschweißen;
- Thermosonic-Schweißen;
- Ultraschallschweißen.

8. Durchführungselement (7) für eine implantierbare Vorrichtung (8), die umfasst:
- ein Substrat (1) mit zumindest einer Durchführungsöffnung (10), wobei die Durchführungsöffnung (10) auf einer ersten Seite des Substrats (1) durch ein Metallkontaktelement (12) mit einer Breite, die größer als die Breite der jeweiligen Durchführungsöffnung (10) ist, hermetisch abgeschlossen ist, und wobei die Durchführungsöffnung mit einem elektrisch leitfähigen Material (11) gefüllt ist;
- eine metallische Zwischenschicht (3), die das Metallkontaktelement (12) an das Substrat (1) um den Umfang der Durchführungsöffnung (10) herum bondet, wobei die metallische Zwischenschicht (10) zumindest eine Bondingschicht (3b) umfasst,
**dadurch gekennzeichnet, dass** die metallische Zwischenschicht (10) des Weiteren zumindest eine Diffusionsbarriereschicht (3a) umfasst.

9. Durchführungselement (7) nach dem vorstehenden Anspruch, wobei das Metallkontaktelement (12) ein Metallfolienkontaktelement ist.

10. Durchführungselement (7) nach Anspruch 8 oder 9, das des Weiteren umfasst:
- ein peripheres Metallelement (24), vorzugsweise ein Metallfolienelement, das von der Peripherie des Substrats (1) vorsteht, das vorzugsweise auf der ersten Seite des Substrats (1) angeordnet ist;
- eine weitere metallische Zwischenschicht (3), die das periphere Metallelement (24) an das Substrat (1) bondet.

11. Durchführungselement (7) nach einem der Ansprüche 8 bis 10, wobei das Metallkontaktelement (12) zumindest eines des Folgenden bildet: eine elektrische Kontaktfläche; eine elektrische Leiterbahn.

12. Durchführungselement (7) nach einem der Ansprüche 8 bis 11, das des Weiteren einen Flansch (36) umfasst, der an der Peripherie des Substrats (1) angebracht ist.

13. Durchführungselement (7) nach einem der Ansprüche 8 bis 12, wobei die metallische Zwischenschicht (3) des Weiteren zumindest eine Absorptionsschicht (3c) umfasst, die so ausgelegt ist, dass sie Laserlicht absorbiert.

14. Durchführungselement (7) nach Anspruch 13, wobei die metallische Zwischenschicht (3) die Abfolge von Schichten Titan - Tantal - Platin umfasst, vorzugsweise die Abfolge von Schichten Titan - Tantal - Platin - Tantal - Titan.

15. Implantierbare Vorrichtung (8), die umfasst:
- ein Durchführungselement (7) nach einem der Ansprüche 8 bis 14;
- eine Abdeckung (2), die direkt oder indirekt an dem Durchführungselement (7) angebracht ist, um einen hermetisch versiegelten Hohlraum zu definieren;
- eine elektronische Vorrichtung (4), die in elektrischer Verbindung mit dem Metallkontaktelement (12) im Hohlraum angeordnet ist.

16. Implantierbare Vorrichtung (8) nach Anspruch 15, wobei die implantierbare Vorrichtung ein MEMS-Mikrophon oder ein MEMS-Drucksensor ist.

17. Verfahren zum Herstellen eines Durchführungselements (7) nach einem der Ansprüche 8 bis 14, das die Schritte umfasst:
- Bereitstellen eines Substrats (1);
- Bilden zumindest einer metallischen Zwischenschicht (3) auf zumindest einem Teil des Substrats (1), wobei die metallische Zwischenschicht (3) zumindest eine Diffusionsbarriereschicht (3a) und zumindest eine Bondingschicht (3b) umfasst;
- Strukturieren des Substrats (3), um zumindest eine Durchführungsöffnung (10) zu bilden;
- Bilden eines Metallkontaktelements (12), um die Durchführungsöffnung (10) zu schließen, wobei das Metallkontaktelement (12) eine Breite aufweist, die größer als die Breite der Durchführungsöffnung (10) ist;
- Füllen der Durchführungsöffnung (10) mit einem elektrisch leitfähigen Material (11),
wobei das Metallkontaktelement (12) mithilfe der metallischen Zwischenschicht (3) an das Substrat (1) gebondet wird, wobei das Bonding durch zumindest eines durchgeführt wird von:
- Diffusionsbonding;
- lasergestütztem Diffusionsbonding;
- Laserschweißen;
- Thermosonic-Schweißen;
- Ultraschallschweißen.

## Revendications

1. Dispositif implantable (8) comprenant :
- un élément de base (1 ; 7) ;
- un élément de recouvrement (2) relié audit élément de base (1 ; 7) afin de définir une cavité prévue pour recevoir un dispositif électronique (4) ;
- une couche intermédiaire métallique (3) placée entre l'élément de base (1) et l'élément de recouvrement (2), et prévue pour relier l'élément de base (1 ; 7) à l'élément de recouvrement (2), ladite couche intermédiaire métallique (3) comprenant au moins une couche de liaison (3b),
**caractérisé en ce que** ladite couche intermédiaire métallique (3) comprend en outre au moins une couche formant barrière de diffusion (3a).

2. Dispositif implantable (8) selon la revendication 1, dans lequel ledit élément de recouvrement est sensiblement transparent.

3. Dispositif implantable (8) selon la revendication 2, comprenant en outre au moins une lentille (82 ; 86) intégrale avec ou reliée audit élément de recouvrement (2).

4. Dispositif implantable (8) selon l'une des revendications 1 à 3, dans lequel ladite couche intermédiaire métallique (3) comprend en outre au moins une couche d'absorption (3c) adaptée pour absorber une lumière laser, ladite couche d'absorption (3c) constituant de préférence une couche d'adhésion.

5. Dispositif implantable (8) selon la revendication 4, dans lequel ladite couche intermédiaire métallique (3) comprend la séquence de couches titane - tantale - platine, de préférence la séquence de couches titane - tantale - platine - tantale - titane.

6. Dispositif implantable (8) selon l'une quelconque des revendications 1 à 5, dans lequel ledit dispositif implantable est un microphone MEMS ou un capteur de pression MEMS.

7. Procédé de fabrication d'un dispositif implantable (8) selon l'une quelconque des revendications 1à 6, comprenant les étapes qui consistent à :
- procurer un élément de base (1 ; 7) comprenant un substrat (1) ;
- procurer un élément de recouvrement (2) ;
- former une couche intermédiaire métallique (3) sur au moins un dudit élément de base et dudit élément de recouvrement, ladite couche intermédiaire métallique (3) comprenant au moins une couche formant barrière de diffusion (3) et au moins une couche de liaison (3b) ;
- relier l'élément de recouvrement (2) à l'élément de base (1 ; 7) à l'aide d'au moins un :
- soudage par diffusion ;
- soudage par diffusion assisté par laser ;
- soudage au laser ;
- soudage thermosonique ;
- soudage par ultrasons.

8. Élément de passage (7) pour un dispositif implantable (8) comprenant :
- un substrat (1) comprenant au moins une ouverture de passage (10), ladite ouverture de passage (10) étant hermétiquement fermée sur un premier côté dudit substrat (1) par un élément de contact métallique (12) ayant une largeur supérieure à la largeur de l'ouverture de passage respective (10), et ladite ouverture de passage étant remplie par un matériau électroconducteur (11) ;
- une couche intermédiaire métallique (3) reliant ledit élément de contact métallique (12) audit substrat (1) autour de la circonférence de ladite ouverture de passage (10), ladite couche intermédiaire métallique (10) comprenant au moins une couche de liaison (3b),
**caractérisé en ce que** ladite couche intermédiaire métallique (10) comprend en outre au moins une couche formant barrière de diffusion (3a).

9. Élément de passage (7) selon la revendication précédente, dans lequel l'élément de contact métallique (12) est un élément de contact à feuille métallique.

10. Élément de passage (7) selon la revendication 8 ou 9, comprenant en outre :
- un élément périphérique métallique (24), de préférence un élément à feuille métallique, dépassant de la périphérie du substrat (1), et de préférence disposé sur ledit premier côté dudit substrat (1) ;
- une autre couche intermédiaire métallique (3) liant l'élément métallique périphérique (24) audit substrat (1).

11. Élément de passage (7) selon l'une quelconque des revendications 8 à 10, dans lequel ledit élément de contact métallique (12) constitue l'un au moins : d'un plot électrique ; d'une piste électrique.

12. Élément de passage (7) selon l'une quelconque des revendications 8 à 11, comprenant en outre un rebord (36) relié à la périphérie du substrat (1).

13. Élément de passage (7) selon l'une quelconque des revendications 8 à 12, dans lequel ladite couche intermédiaire métallique (3) comprend en outre au moins une couche d'absorption (3c) conçue pour absorber la lumière.

14. Élément de passage (7) selon la revendication 13, dans lequel ladite couche intermédiaire métallique (3) comprend la séquence de couches titane - tantale - platine, de préférence la séquence de couches titane - tantale - platine -tantale - titane.

15. Dispositif implantable (8) comprenant :
- un élément de passage (7) selon l'une quelconque des revendications 8 à 14 ;
- un élément de recouvrement (2) relié directement ou indirectement audit élément de passage (7) afin de définir une cavité hermétiquement fermée ;
- un dispositif électronique (4) disposé dans ladite cavité en liaison électrique avec ledit élément de contact métallique (12).

16. Dispositif implantable (8) selon la revendication 15, dans lequel ledit dispositif implantable est un microphone MEMS ou un capteur de pression MEMS.

17. Procédé de fabrication d'un élément de passage (7) selon l'une quelconque des revendications 8 à 14, comprenant les étapes qui consistent à :
- procurer un substrat (1);
- former au moins une couche intermédiaire métallique (3) sur au moins une partie dudit substrat (1), ladite couche intermédiaire métallique (3) comprenant au moins une couche formant barrière de diffusion (3a) et au moins une couche de liaison (3b) ;
- structurer ledit substrat (3) afin de former au moins une ouverture de passage (10) ;
- former un élément de contact métallique (12) afin de fermer ladite ouverture de passage (10), ledit élément de contact métallique (12) ayant une largeur supérieure à la largeur de ladite ouverture de passage (10) ;
- remplir ladite ouverture de passage (10) avec un matériau électroconducteur (11),
dans lequel ledit élément de contact métallique (12) est relié au substrat (1) grâce à ladite couche intermédiaire métallique (3), ladite liaison étant réalisée par au moins un :
- soudage par diffusion ;
- soudage par diffusion assisté par laser ;
- soudage au laser ;
- soudage thermosonique ;
- soudage ultrasonique.
